(19) Europäisches Patentamt
European Patent Office
Office européen des brevets

(11) EP 1 859 797 A1

(12) EUROPEAN PATENT APPLICATION
published in accordance with Art. 158(3) EPC

(43) Date of publication:
**28.11.2007 Bulletin 2007/48**

(21) Application number: **06715262.9**

(22) Date of filing: **28.02.2006**

(51) Int Cl.:
***A61K 31/404** (2006.01)*
***A61K 31/18** (2006.01)*
***A61K 31/343** (2006.01)*
***A61K 31/381** (2006.01)*
***A61K 31/4741** (2006.01)*
***A61K 31/498** (2006.01)*
***A61K 31/7068** (2006.01)*
***A61K 33/24** (2006.01)*
***A61K 45/00** (2006.01)*
***A61P 9/00** (2006.01)*
***A61P 35/00** (2006.01)*

(86) International application number:
**PCT/JP2006/304219**

(87) International publication number:
**WO 2006/090931 (31.08.2006 Gazette 2006/35)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.02.2005 JP 2005055132**

(71) Applicant: **Eisai R&D Management Co., Ltd.**
**Tokyo 112-8088 (JP)**

(72) Inventors:
- **OWA, Takashi**
  **Tsukuba-shi**
  **Ibaraki 3002635 (JP)**
- **OZAWA, Yoichi**
  **Tsukuba-shi**
  **Ibaraki 3002635 (JP)**
- **SEMBA, Taro**
  **Tsukuba-shi, Ibaraki 3002635 (JP)**
- **HATA, Naoko**
  **Tsukuba-shi, Ibaraki 3002635 (JP)**

(74) Representative: **Woods, Geoffrey Corlett et al**
**J.A. KEMP & CO.**
**Gray's Inn**
**14 South Square**
**London WC1R 5JJ (GB)**

# (54) NOVEL CONCOMITANT USE OF SULFONAMIDE COMPOUND WITH ANTI-CANCER AGENT

(57) The present invention relates to a pharmaceutical composition, a kit, a method of treating cancer and/or a method of inhibiting angiogenesis comprising a sulfonamide compound in combination with a platinum complex, a DNA-topoisomerase I inhibitor, an antimetabolite, a microtubule inhibitor or an antibiotic.

EP 1 859 797 A1

## Description

FIELD OF THE INVENTION

The present invention relates to a novel pharmaceutical composition, a kit, a method for treating cancer and/or a method for inhibiting angiogenesis, characterized by comprising a sulfonamide compound in combination with at least one substance selected from the group consisting of a platinum complex such as Oxaliplatin and Cisplatin, a DNA-topoisomerase I inhibitor such as CPT-11, an antimetabolite such as Gemcitabine and Methotrexate, a microtubule inhibitor such as Paclitaxel and an antibiotic such as Doxorubicin.

BACKGROUND OF THE INVENTION

Examples of conventionally used chemotherapy drugs for cancer include alkylating agents such as cyclophosphamide, antimetabolites such as methotrexate and fluorouracil, antibiotics such as adriamycin, mitomycin, bleomycin, plant-derived taxol, vincristine and etoposide, and metal complexes such as cisplatin. All of them, however, have not been sufficient in anti-tumor effects, and thus there has been a strong need for development of a novel anti-tumor agent.

Recently, a sulfonamide compound has been reported as a useful anti-tumor agent[1-4]. In particular, N-(3-cyano-4-methyl-1H-indole-7-yl)-3-cyanobenzenesulfonamide (hereinafter, also referred to as "E7820"), N-[[(4-chlorophenyl)amino]carbonyl]-2,3-dihydro-1H-indene-5-sulfonamide (hereinafter, also referred to as "LY186641"), N-[[(3,4-dichlorophenyl)amino]carbonyl]-2,3-dihydrobenzofuran-5-sulfonamide (hereinafter, also referred to as "LY295501"), N-(2,4-dichlorobenzoyl)-4-chlorophenylsulfonamide (hereinafter, also referred to as "LY-ASAP"), N-(2,4-dichlorobenzoyl)-5-bromothiophene-2-sulfonamide (hereinafter, also referred to as "LY573636") and 2-sulfanylamide-5-chloroquinoxaline (hereinafter, also referred to as "CQS") are active against various types of tumors and thus are very useful.

The presence and the kind of effects resulting from combining these compounds, however, have not been reported so far.

Recently, methods were established for simultaneously detecting expression levels of multiple genes using various DNA microarrays. Thus, DNA microarrays have been used for wide-ranging purposes[5 and 6]. In addition, several reports have been made about using DNA microarrays (In part, rhere is a macroarray using membrane filters) for examining changes in gene expressions upon use of anti-cancer drugs against tumor cells[7-9]. These reports show that the analysis of gene expression variability is highly useful in comprehensively studying the characteristic comparison among a plurality of cell populations, the biological changes in cells caused by treatment of drug or the like at molecular level.

Furthermore, reports have also been made to the analysis of gene expression profiles of 60 types of cancer cell line panels from the US National Cancer Institute for reclassification of these cell lines and examination of their characteristics[10], and to discussion regarding relationship among the gene expression profiles of these 60 types of cancer cell line panels and sensitivity of each cell line to various anti-cancer drugs[11].

References

(1) International Publication No. WO00/50395.
(2) European Patent Publication No. 0222475.
(3) International Publication No. WO02/098848.
(4) International Publication No. WO03/035629.
(5) Schena M, Shalon D, Davis RW, Brown PO. Science, 1995, 270, 467-70.
(6) Lockhart, D.J., Dong, H., Byrne, M.C., Follettie, M.T., Gallo, M.V., Chee, M.S., Mittmann, M., Wang C., Kobayashi, M., Horton, H. Brown, E.L., Nature Biotechnology, 1996, 14, 1675-1680.
(7) Rhee CH, Ruan S, Chen S, Chenchik A, Levin VA, Yung AW, Fuller GN, Zhang W, Oncol Rep, 1999, 6, 393-401.
(8) Zimmermann J, Erdmann D, Lalande I, Grossenbacher R, Noorani M, Furst P, Oncogene, 2000, 19, 2913-20.
(9) Kudoh K, Ramanna M, Ravatn R, Elkahloun AG, Bittner ML, Meltzer PS, Trent JM, Dalton WS, Chin KV, Cancer Res, 2000, 4161-6.
(10) Ross DT, Scherf U, Eisen MB, Perou CM, Rees C, Spellman P, Iyer V, Jeffrey SS, Van de Rijn M, Waltham M, Pergamenschikov A, Lee JC, Lashkari D, Shalon D, Myers TG, Weinstein JN, Botstein D, Brown PO, Nat Genet, 2000, 24, 227-35.
(11) Scherf U, Ross DT, Waltham M, Smith LH, Lee JK, Tanabe L, Kohn KW, Reinhold WC, Myers TG, Andrews

DT, Scudiero DA, Eisen MB, Sausville EA, Pommier Y, Botstein D, Brown PO, Weinstein JN, Nat Genet, 2000, 24, 236-44.

DISCLOSURE OF THE INVENTION

The present invention was achieved regarding the circumstances described above. The problem to be solved by the invention is to find a pharmaceutical composition and a kit having a remarkable anti-tumor activity and/or angiogenesis inhibiting activity, and a method for treating cancer and/or a method for inhibiting angiogenesis.

In order to solve the above problem, the present inventors have gone through keen examination, as a result of which combinational use of E7820 and Taxol (Paclitaxel), SN38 (active form of CPT-11), Methotrexate, Cisplatin, Gemcitabine or Doxorubicin was found to show a statistically significant (by combination index) synergistic antiproliferative effect in a vascular endothelial cell proliferation assay *(in vitro).* In addition, combinational use of E7820 and Paclitaxel, SN38 (active form of CPT-11), Cisplatin, Gemcitabine or Doxorubicinin was found to show a statistically significant (by combination index) synergistic effect on inhibition of lumen formation in a vascular endothelial cell lumen formation assay *(in vitro).* Combinational use of E7820 and Oxaliplatin or CPT-11 was found to show a statistically significant (by two-way ANOVA) synergistic anti-tumor effect in a subcutaneous transplant model *(in vivo)* of human colon cancer cell line. Moreover, combinational use of E7820 and Gemcitabine was found to show a statistically significant (by two-way ANOVA) synergistic anti-tumor effect in a subcutaneous transplant model *(in vivo)* of human pancreas cancer cell line. Combinational use of E7820 and at least one compound selected from the group consisting of Oxaliplatin, CPT-11 and Gemcitabine showed a remarkable anti-tumor effect that cannot be seen with a compound selected from the group consisting of Oxaliplatin, CPT-11 and Gemcitabine alone.

In experiments using DNA microarrays and cancer cell line panels, genetic alteration patterns and antiproliferative activities of E7070 (which refers to "N-(3-chloro-1H-indole-7-yl)-4-sulfamoylbenzenesulfonamide" in this specification), E7820, LY186641, LY295501, LY573636, CQS and combinations thereof were found to show high correlation. In an assay for determining antiproliferative activity, a cancer cell line resistant to E7070 was found to show cross-resistance to E7820, LY186641, LY295501, LY-ASAP, LY573636 or CQS. From these results, the present inventors have found that E7070, E7820, LY186641, LY295501, LY-ASAP, LY573636, CQS and combinations thereof have the same or similar action mechanisms that result in the same or similar gene alterations and effects.

Accordingly, E7820, LY186641, LY295501, LY-ASAP, LY573636, CQS or a combination thereof is considered to show a good anti-tumor activity and/or angiogenesis inhibiting activity when used in combination with at least one compound selected from the group consisting of Oxaliplatin, Cisplatin, CPT-11, Gemcitabine, Methotrexate, Paclitaxel and Doxorubicin, and thus a combination of a sulfonamide compound, preferably E7820, LY186641, LY295501, LY-ASAP, LY573636, CQS or a combination thereof, and at least one substance selected from the group consisting of Oxaliplatin, Cisplatin, CPT-11, Gemcitabine, Methotrexate, Paclitaxel and Doxorubicin can be used as a useful pharmaceutical composition or a kit, which can be used for treatment of cancer and/or inhibition of angiogenesis.
Thus, the present invention relates to:

(1) A pharmaceutical composition comprising a sulfonamide compound in combination with and at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof.

(2) A kit comprising:

(a) at least one selected from the group consisting of a packaging container, an instruction and a package insert describing the combinational use of a sulfonamide compound and at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof; and
(b) a pharmaceutical composition comprising the sulfonamide compound.

(3) A kit comprising a set of a formulation comprising a sulfonamide compound and a formulation comprising at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof.

(4) Use of a sulfonamide compound for producing a pharmaceutical composition in combination with at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof.

(5) A method for treating cancer and/or a method for inhibiting angiogenesis comprising administering a sulfonamide compound and at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof to a patient.

(6) A pharmaceutical composition comprising a sulfonamide compound for administering to a patient in combination with at least one substance selected from the group consisting of a platinum complex, a DNA-topoisomerase I inhibitor, an antimetabolite, a microtubule inhibitor and an antibiotic.

Examples of the sulfonamide compounds according to (1)-(6) above include N-(3-cyano-4-methyl-1H-indole-7-yl)-3-cyanobenzenesulfonamide, a pharmacologically acceptable salt thereof or a solvate thereof.

In addition, the sulfonamide compounds according to (1)-(6) above include at least one compound selected from the group consisting of:

a compound represented by General Formula (I)

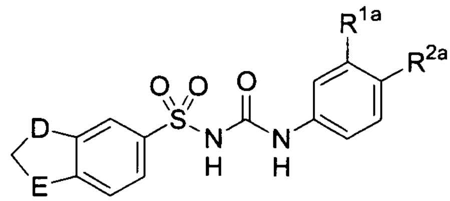

(I)

[wherein, E represents -O-, $-N(CH_3)-$, $-CH_2-$, $-CH_2CH_2-$ or $-CH_2O-$ D represents $-CH_2-$or -O-, $R^{1a}$ represents a hydrogen atom or a halogen atom, and $R^{2a}$ represents a halogen atom or a trifluoromethyl group];

a compound represented by General Formula (II)

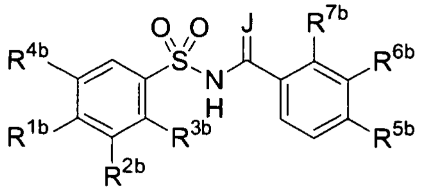

(II)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom),$R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];

a compound represented by Formula (III)

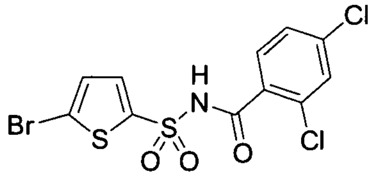

(III)

;

and

a compound represented by Formula (IV)

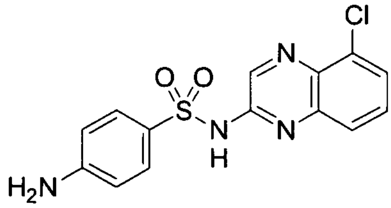

(IV)

or a pharmacologically acceptable salt thereof, or a solvate thereof.

In (1)-(6) above, the group consisting of a platinum complex, a DNA-topoisomerase I inhibitor, an antimetabolite, a microtubule inhibitor and an antibiotic may be a group consisting of Oxaliplatin, Cisplatin, CPT-11, Gemcitabine, Methotrexate, Paclitaxel and Doxorubicin.

The present invention provides a pharmaceutical composition and a kit that show a remarkable anti-tumor activity and/or angiogenesis inhibiting activity, and a method for treating cancer and/or a method for inhibiting angiogenesis.

More specifically, the present invention provides a pharmaceutical composition and a kit that show a remarkable anti-tumor activity and/or angiogenesis inhibiting activity and a method for treating cancer and/or a method for inhibiting angiogenesis, by combining a sulfonamide compound, that is, at least one compound selected from (A) E7820, (B) a compound represented by General Formula (I), preferably LY186641 or LY295501, (C) a compound represented by General Formula (II), preferably LY-ASAP, (D) LY573636 and (E) CQS, with at least one substance selected from (i) a platinum complex, preferably Oxaliplatin or Cisplatin, (ii) a DNA-topoisomerase I inhibitor, preferably CPT-11, (iii) an antimetabolite, preferably Gemcitabine or Methotrexate, (iv) a microtubule inhibitor, preferably Paclitaxel and (v) an antibiotic, preferably Doxorubicin. Thus, the pharmaceutical composition, the kit and the methods of the invention can be used for treating cancer or inhibiting angiogenesis.

## BRIEF DESCRIPTION OF THE DRAWINGS

Figure 1 shows an effect on tumor growth inhibition obtained by combinational use of E7820 and oxaliplatin in a subcutaneous transplant model *(in vivo)* of human colon cancer cell line (Colo320DM). In Figure 1, * indicates a statistically significant synergistic effect at a significance level of less than 0.05. In Figure 1, Day# indicates days from the first day of administration (Day 1).

Figure 2 shows an effect on tumor growth inhibition obtained by combinational use of E7820 and CPT-11 in a subcutaneous transplant model *(in vivo)* of human colon cancer cell line (Colo320DM). In Figure 2, * indicates a statistically significant synergistic effect at a significance level of less than 0.01. In Figure 2, Day# indicates days from the first day of administration (Day 1).

Figure 3 shows an effect on tumor growth inhibition obtained by combinational use of E7820 and Gemcitabine in a subcutaneous transplant model *(in vivo)* of human pancreas cancer cell line (KP-1). In Figure 3, * indicates a statistically significant synergistic effect at a significance level of less than 0.01. In Figure 3, Day# indicates days from the first day of administration (Day 1).

Figure 4 shows the results of hierarchical cluster analysis in the DNA microarrays in Example 7.

Figure 5 shows correlation coefficients in the DNA microarrays in Example 8.

Figure 6 shows the results of hierarchical cluster analysis of the DNA microarrays in Example 8.

Figure 7 shows correlation coefficients in the DNA microarrays in Example 8.
Figure 8 shows the results of hierarchical cluster analysis in the DNA microarrays in Example 8.
Figure 9 shows antiproliferative effects of E7070, E7820, CQS, LY186641, LY295501 and LY-ASAP on HCT116-C9, HCT116-C9-C1 and HCT116-C9-C4 as measured by cell growth inhibition assay.
Figure 10 shows antiproliferative effects of E7070 and LY573636 on HCT116-C9, HCT116-C9-C1 and HCT116-C9-C4 as measured by cell growth inhibition assay.

BEST MODES FOR CARRYING OUT THE INVENTION

Hereinafter, embodiments of the present invention will be described. The following embodiments are described for illustrating the present invention and they are not intended to limit the present invention. The present invention may be carried out in various embodiments as long as it does not depart from the scope of the invention.
The publications, laid-open patent publications, patent publications and other patent documents cited herein are incorporated herein by reference.

1. Sulfonamide compound

A pharmaceutical composition and/or a kit and a method for treating cancer and/or a method for inhibiting angiogenesis of the present invention comprise a sulfonamide compound.
According to the present invention, the sulfonamide compound comprises a compound represented by the following General Formula (V).

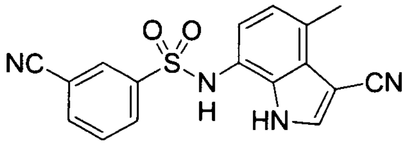
(V)

**E7820**

The compound represented by Formula (V) is N-(3-cyano-4-methyl-1H-indole-7-yl)-3-cyanobenzenesulfonamide and may also be referred to as E7820.

E7820 can be produced according to a known method, for example, by a method described in International Publication No. 00/50395 (pamphlet) (WOOO/50395).

According to the present invention, the sulfonamide compound comprises a compound represented by the following General Formula (I).

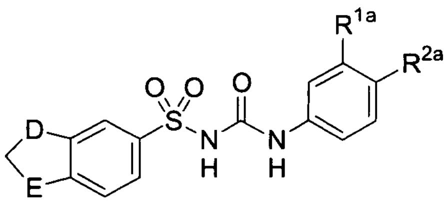
(I)

In General Formula (I) above, E represents -O-, -N($CH_3$)-, -$CH_2$-, -$CH_2CH_2$- or -$CH_2O$-, D represents -$CH_2$- or -O-, $R^{1a}$ represents a hydrogen atom or a halogen atom (e.g., a fluorine atom, a chlorine atom, a bromine atom or an iodine atom), and $R^{2a}$ represents a halogen atom or a trifluoromethyl group.
The compound represented by General Formula (I) of the invention can be produced according to a known method, for example, by a method described in European Patent Publication No. 0222475A1 (specification) (EP0222475A1).
In General Formula (I), a preferable compound is LY186641 or LY295501.
LY186641 is
N-[[(4-chlorophenyl)amino]carbonyl]-2,3-dihydro-1H-indene-5-sulfonamide, whose structural formula is represented by

the following Formula (VI).

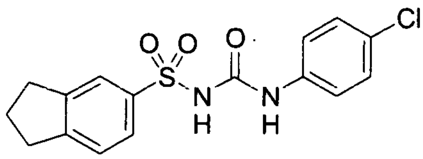

(VI)

LY186641

LY186641 can be produced according to a known method, for example, by a method described in European Patent Publication No. 0222475A1 (specification) (EP0222475A1).

According to the present invention, LY295501 is N-[[(3,4-dichlorophenyl)amino]carbonyl]-2,3-dihydrobenzofuran-5-sulfonamide, whose structural formula is represented by the following Formula (VII).

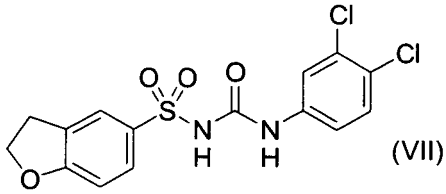

(VII)

LY295501

LY295501 can be produced according to a known method, for example, by those described in European Patent Publication No. 0222475A1 (specification) (EP0222475A1) and/or European Patent Publication No. 0555036A2 (specification) (EP0555036A2).

Furthermore, according to the present invention, the sulfonamide compound comprises a compound represented by the following General Formula (II).

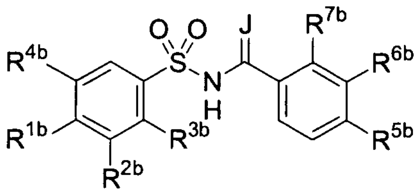

(II)

In General Formula (II), J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$, represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ refers to a hydrogen atom, a halogen atom, an optionally substituted $C_1$-$C_6$ alkyl group, $-CF_3$ or a nitro group, $R^{6b}$ refers to a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ refers to a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom).

In General Formula (II), a "halogen atom" is preferably a fluorine atom, a chlorine atom, a bromine atom or an iodine atom.

In General Formula (II), "$C_1$-$C_6$ alkyl group" refers to linear or branched alkyl group with a carbon number of 1-6, and includes, but not limited to, methyl group, ethyl group, n-propyl group, isopropyl group, n-butyl group, isobutyl group, sec-butyl group, tert-butyl group, n-pentyl group (amyl group), isopentyl group, neopentyl group, tert-pentyl group, 1-methylbutyl group, 2-methylbutyl group, 1,2-dimethylpropyl group, n-hexyl group, isohexyl group, 1-methylpentyl group, 2-methylpentyl group, 3-methylpentyl group, 1-ethylpropyl group, 1,1-dimethylbutyl group, 1,2-dimethylbutyl group, 2,2-dimethylbutyl group, 1,3-dimethylbutyl group, 2,3-dimethylbutyl group, 3,3-dimethylbutyl group, 1-ethylbutyl group, 2-ethylbutyl group, 1,1,2-trimethylpropyl group, 1,2,2-trimethylpropyl group, 1-ethyl-1-methylpropyl group and 1-ethyl-2-methylpropyl group. Examples of preferable groups among these include methyl group, ethyl group, n-propyl group, isopropyl group, n-butyl group, isobutyl group, sec-butyl group, tert-butyl group, n-pentyl group and n-hexyl group.

In General Formula (II), "$C_1$-$C_4$ alkoxy group" refers to alkoxy group with a carbon number of 1-4, and preferably includes, but not limited to, methoxy group, ethoxy group, n-propoxy group, isopropoxy group, n-butoxy group, isobutoxy group, sec-butoxy group and tert-butoxy group.

In General Formula (II), examples of alkyl group of "$C_1$-$C_4$ alkylthio group" include, but not limited to, methyl, ethyl, n-propyl, isopropyl, n-butyl, isobutyl, sec-butyl and tert-butyl.

In General Formula (II), examples of "$C_1$-$C_4$ alkoxy carbonyl group" include, but not limited to, methoxy carbonyl group, ethoxy carbonyl group, n-propoxy carbonyl group, isopropoxy carbonyl group, n-butoxy carbonyl group, isobutoxy carbonyl group, sec-butoxy carbonyl group and tert-butoxy carbonyl group. In General Formula (II), examples of substituents to be introduced include, but not limited to, substituents such as $C_1$-$C_6$ alkyl group (e.g., methyl group, ethyl group, n-propyl group, isopropyl group, n-butyl group, isobutyl group, sec-butyl group, tert-butyl group, etc.), $C_1$-$C_4$ alkoxy group (e.g., methoxy group, ethoxy group, n-propoxy group, isopropoxy group, n-butoxy group, isobutoxy group, sec-butoxy group, tert-butoxy group, etc.), amino group, hydroxyl group, a halogen atom (e.g., a fluorine atom, a chlorine atom, a bromine atom or an iodine atom) and silyl group.

The compound represented by General Formula (II) of the invention can be produced by a known method such as the method described in International Publication No. 02/098848 (pamphlet) (WO02/098848).

In General Formula (II), a preferable compound is LY-ASAP.

LY-ASAP is N-(2,4-dichlorobenzoyl)-4-chlorophenylsulfonamide, whose structural formula is represented by the following Formula (VIII).

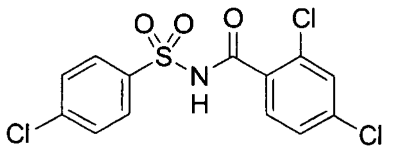

(VIII)

**LY-ASAP**

LY-ASAP can be produced by a known method such as the method described in International Publication No. 02/098848 (pamphlet) (WO02/098848).

According to the present invention, an example of the sulfonamide compound includes LY573636. According to the invention, LY573636 is N-(2,4-dichlorobenzoyl)-5-bromothiophene-2-sulfonamide, whose structural formula is represented by the following Formula (III).

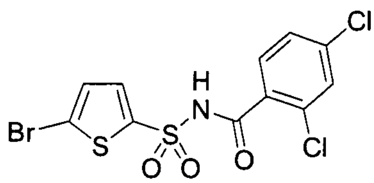

(III)

LY573636

LY573636 is preferably a sodium salt.

LY573636 can be produced by a known method. For example, it can be produced in the same manner as the method described in International Publication No. 02/098848 (pamphlet) (WO02/098848) using commercially available 5-bromothiophene-2-sulfonyl chloride and 2,4-dichlorobenzoic acid.

LY573636 can be produced by a method described in Example 63 of International Publication No. 03/035629

(pamphlet) (WO03/035629).

According to the present invention, an example of the sulfonamide compound includes CQS. According to the present invention, CQS is 2-sulfanylamide-5-chloroquinoxaline, whose structural formula is represented by the following Formula (IV).

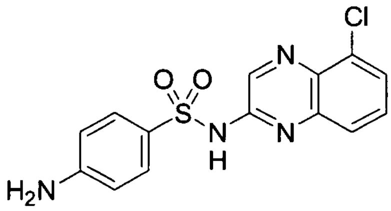

(IV)

CQS

CQS can be produced according to a known method, for example, by the method according to (J. Am. Chem. Soc., 1947, 71, 6-10).

The sulfonamide compound may form a pharmacologically acceptable salt with acid or base. The sulfonamide compound of the invention also comprises these pharmacologically acceptable salts. Examples of salts formed with acids include inorganic acid salts such as hydrochloride salts, hydrobromide salts, sulfate salts and phosphate salts, and salts formed with organic acids such as formic acid, acetic acid, lactic acid, succinic acid, fumaric acid, maleic acid, citric acid, tartaric acid, benzoic acid, methanesulfonic acid, benzenesulfonic acid, p-toluenesulfonic acid and trifluoroacetic acid. Examples of salts formed with bases include alkali metal salts such as sodium salt and potassium salt, alkaline earth metal salts such as calcium salt and magnesium salt, salts with organic bases such as trimethylamine, triethylamine, pyridine, picoline, dicyclohexylamine, N,N'-dibenzylethylenediamine, arginine and lysine (organic amine salts), and ammonium salts.

Furthermore, the sulfonamide compound may be an anhydride, and may form a solvate such as a hydrate. The solvate may be either a hydrate or a nonhydrate, preferably a hydrate. The solvent used may be water, alcohol (e.g., methanol, ethanol or n-propanol), dimethylformamide or the like.

If solvates and/or enantiomers of these compounds exist, the sulfonamide compound of the invention comprises these solvates and/or enantiomers. The sulfonamide compound of the invention also comprises sulfonamide compounds that undergo metabolism *in vivo* such as oxidation, reduction, hydrolysis and conjugation. Moreover, the sulfonamide compound of the invention also comprises compounds that generate sulfonamide compounds by undergoing metabolism such as oxidation, reduction and hydrolysis *in vivo.*

2. Platinum complex, DNA-topoisomerase I inhibitor, antimetabolite, microtubule inhibitor and antibiotic

A pharmaceutical composition and/or a kit and a method for treating cancer and/or a method for inhibiting angiogenesis of the invention comprise at least one substance selected from the group consisting of a platinum complex, a DNA-topoisomerase I inhibitor, an antimetabolite, a microtubule inhibitor and an antibiotic.

(1) Platinum complexes

According to the present invention, a platinum complex may be a complex having platinum as a central metal (platinum complex) and is not limited by the degree of bond between the central metal and the ligand, charge of the complex, increase in the number of central metals due to the bridged structure of the ligand, or the like. According to the present invention, the platinum complex may be a platinum formulation obtained by formulating a platinum complex. According to the present invention, the platinum complex may also be referred to as a platinum formulation.

According to the present invention, examples of the platinum complex include Oxaliplatin, Carboplatin, Cisplatin (CDDP), Lobaplatin, AR-726, Miriplatin, Picoplatin, PLD-147, Satraplatin, Thioplatin and Triplatin, preferably Oxaliplatin or Cisplatin, more preferably Oxaliplatin. The platinum complex may be produced by a known method or may be purchased.

According to the present invention, Oxaliplatin refers to oxalato (1R, 2R-cyclohexanediamine) platinum and is a compound represented by Formula (IX).

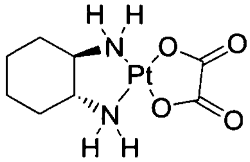

(IX)

Oxaliplatin can be produced by a known method. Alternatively, Oxaliplatin is available by purchasing Eloxatin® from Sanofi Aventis.
According to the present invention, Carboplatin refers to cis-diammine(1, 1-cyclobutanedicarboxylate) platinum.
According to the present invention, Carboplatin is available by purchasing Paraplatin (Bristol).
According to the present invention, Cisplatin (CDDP) refers to cis-diamminedichloroplatinum II and is a compound represented by Formula (XX).

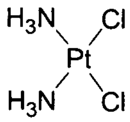
(XX)

According to the present invention, Cisplatin (CDDP) is available by purchasing Randa (Nippon Kayaku) or Briplatin (Bristol).
According to the present invention, Lobaplatin refers to [SP-4-3-(S),(trans)]-(1,2-cyclobutanedimethanamine-N,N'-)[2-hydroxypropanoate(2-)-O 1,02]-platinum.
Lobaplatin may be produced by a known method (DE4115559).
According to the present invention, AR-726 refers to cis-bis-neodecanoate-trans-R, R-1,2-diamincyclohexane-Pt(II).
AR-726 may be produced by a known method.
According to the present invention, Miriplatin refers to (SP-4-2)-[(1R,2R)-cyclohexane-1,2-diamine-N,N']bis (tetradecanoato-O)platinum.
Miriplatin may be produced by a known method (EP193936).
According to the present invention, Picoplatin refers to (SP-4-3)-amminedichloro(2-methylpyridine)platinum.
Picoplatin may be produced by a known method.
According to the present invention, PLD-147 refers to (OC-6-43)-bis(acetato)(1-adamantylamine)ammine-dichloro-platinum (IV).
PLD-147 may be produced by a known method (US6503943).
According to the present invention, Satraplatin refers to (OC-6-43)-bis(acetato)amminedichloro(cyclohexylamine)platinum.
Satraplatin may be produced by a known method (EP328274).
According to the present invention, Thioplatin refers to bis-(O-ethyldithiocarbamato)platinum (II).
Thioplatin may be produced by a known method (WO00/10543).
According to the present invention, Triplatin refers to trans-[bis[trans-diamminechloroplatinum(p-hexane-1,6-diamine)]]diammineplatinum.
Triplatin may be produced by a known method (US5744497).

(2) DNA-topoisomerase I inhibitors

According to the present invention, a DNA-topoisomerase I inhibitor refers to a substance having an effect of inhibiting DNA-topoisomerase I.
According to the present invention, examples of a DNA-topoisomerase I inhibitor include CPT-11, Topotecan hydrochloride, Exatecan, Rubitecan, 9-amino-camptothecin, Lurtotecan dihydrochloride, Gimatecan and Edotecarin, preferably CPT-11.
A DNA-topoisomerase I inhibitor may be produced by a known method or may be purchased.
According to the present invention, CPT-11 refers to irinotecan hydrochloride trihydrate ([1,4'-Bipiperidine]-1'-carboxylic acid (S)-4,11-diethyl-3,4,12,14-tetrahydro-4-hydroxy-3,14-dioxo-IH-pyrano-[3',4':6,7]-indoli zino[1,2-b] quinolin-9-yl ester Hydrochloride trihydrate) and is a compound represented by Formula (X).

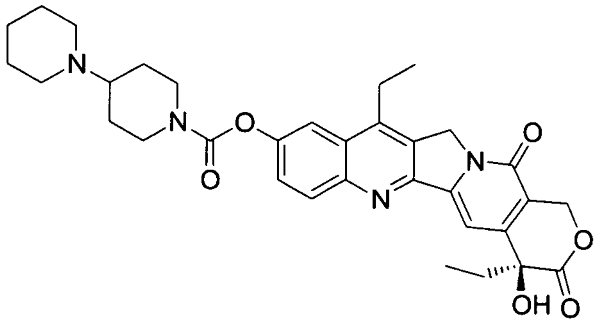

HCl $3H_2O$

(X)

CPT-11 may be produced by a known method. CPT-11 is also available by purchasing Topotecin® from Daiichi Pharm.

According to the present invention, SN38, i.e., an active form of CPT-11, may be used as the DNA-topoisomerase I inhibitor. SN38 refers to 7-ethyl-10-hydro-(20)S-Camptothecin. SN38 is available by purchasing from ABATRA.

According to the present invention, Topotecan hydrochloride refers to (4S)-10-[(dimethylamino)methyl]-4-ethyl-4, 9-dihydroxy-1H-pyrano[3',4':6,7]indolizino [1,2-b] quinoline-3,14 (4H,12H)-dione hydrochloride, and is a compound represented by Formula (XI).

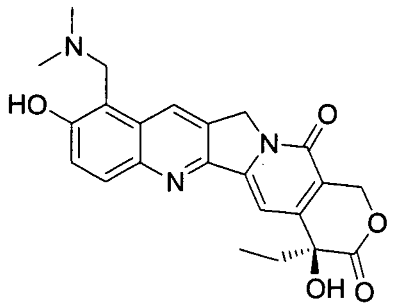

HCl

(XI)

Topotecan hydrochloride may be produced by a known method (US Patent No. 5004758 specification (US5004758)). Topotecan hydrochloride is available by purchasing Hycamtin® from Nippon Kayaku.

According to the present invention, Exatecan refers to (1S,9S)-1-amino-9-ethyl-5-fluoro-1,2,3,9,12,15-hexahydro-9-hydroxy-4-methyl-10H, 13H-benzo[de]pyrano[3',4':6,7]indolizino[1,2-b]quinoline-10,13-dione and is a compound represented by Formula (XII).

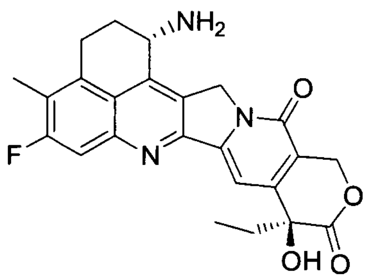

(XII)

Exatecan may be produced by a known method (Japanese Laid-Open Patent Publication No. 05-59061 (JP93-59061)).

According to the present invention, Rubitecan refers to (4S)-ethyl-4-hydroxy-10-nitro-1, 12-dihydro-14H-pyrano [3',4':6,7]-indolizino[1,2-b]quinoline-3,14(4H, 12H)-dione and is a compound represented by Formula (XIII).

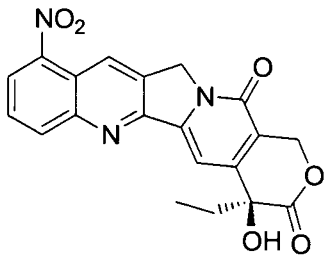

(XIII)

Rubitecan may be produced by a known method (Journal of Medicinal Chemistry (1986), 29(11), 2358-63 and Japanese Laid-Open Patent Publication No. 59-051288).

According to the present invention, 9-amino-camptothecin refers to (4S)-ethyl-4-hydroxy-10-amino-1,12-dihydro-14H-pyrano[3',4':6,7]-indolizino[1,2-b]qui noline-3, 14(4H,12H)-dione, and is a compound represented by Formula (XIV).

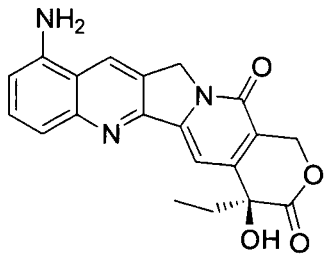

(XIV)

9-amino-camptothecin may be produced by a known method (Japanese Laid-Open Patent Publication No. 59-051289).

According to the present invention, Lurtotecan dihydrochloride refers to 7-(4-methylpiperazinomethylene)-10,11-ethylenedioxy-20(s)-camptothecin dihydrochloride and is a compound represented by Formula (XV)

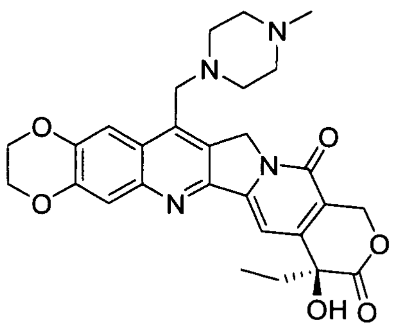

2HCl

(XV)

Lurtotecan dihydrochloride may be produced by a known method (WO95/29919).

According to the present invention, Gimatecan refers to (4S)-11-[(E)-[[1,1-dimethylethoxy]imino]methyl]-4-ethyl-4-hydroxy-1, 12-dihydro-14H-pyrano[3',4':6,7]-indolizino[1,2-b]quinoline-3,14(4H)-dione and is a compound represented by Formula (XVI).

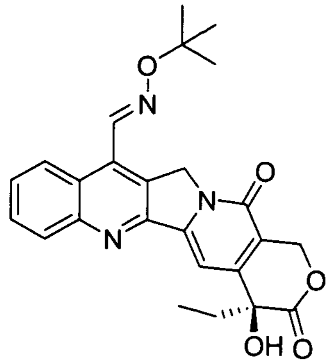

(XVI)

Gimatecan may be produced by a known method (WO00/053607).

According to the present invention, Edotecarin refers to 12-β-D-glucopyranosyl-2,10-dihydroxy-6-[[2-hydroxy-1-(hydroxymethyl)ethyl]amino]-1 2,13-dihydro-6H-indolo[2,3-a]pyrrole[3,4-c]carbazole-5,7-dione, and is a compound represented by Formula (XVII).

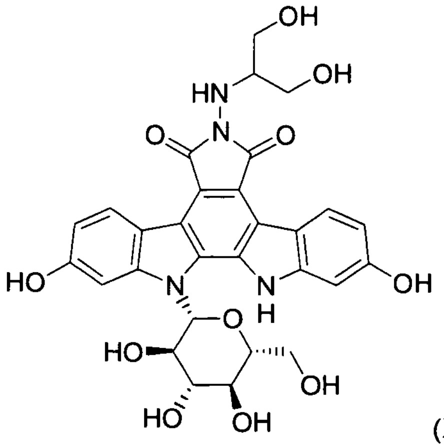

(XVII)

Edotecarin may be produced by a known method (WO95/30682).

(3) Antimetabolites

According to the present invention, an antimetabolite is a compound that has a similar structure to a substance necessary for a cell metabolism such as nucleic acid synthesis or proteosynthesis. Accordingly, it inhibits the cell metabolism by this structural similarity.

An example of the antimetabolite of the invention includes a Cytidine derivative, specific examples being Gemcitabine, Cytarabine (araC), Enocitabine, Cytarabine ocfosfate, 5-azacytidine and CNDAC, preferably Gemcitabine.

In addition, an example of the antimetabolite of the invention includes an antifolate drug, a specific example being Methotrexate. An antifolate drug inhibits nucleic acid synthesis by inhibiting dihydrofolate reductase.

An antimetabolite may be produced by a known method or it can be purchased.

According to the present invention, Gemcitabine refers to gemcitabine hydrochloride (2'-deoxy-2',2'-difluorocytidine hydrochloride) and is a compound represented by Formula (XVIII).

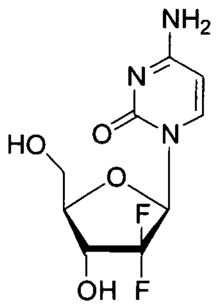

HCl

(XVIII)

Gemcitabine may be produced by a known method. Gemcitabine is also available by purchasing GEMZAR® from Eli Lilly Japan.

Cytarabine (araC) is available by purchasing Cylocide (Nippon Shinyaku) or Cylocide N (Nippon Shinyaku).

Enocitabine (BH-AC) is available by purchasing Sunrabin (Asahi Kasei Pharma).

Cytarabine ocfosfate (SPAC) is available by purchasing Starasid (Nippon Kayaku).

5-azacytidine may be produced by a known method and is available by purchasing from Nacalai Tesque, Inc.

According to the present invention, CNDAC refers to 1-(2-C-cyano-2-deoxy-β-D-arabino-pentofuranosyl)-N-palmitoylcytosine and is a compound represented by Formula (XIX).

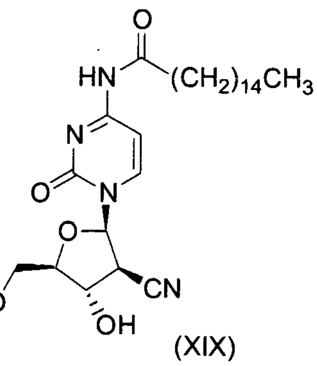

(XIX)

CNDAC may be produced by a known method (EP536939).

According to the present invention, Methotrexate refers to N-{4-[N-(2,4-diaminopteridin-6-ylmethyl)-N-methylamino]benzoyl}-L-glutamic acid and is a compound represented by Formula (XXI).

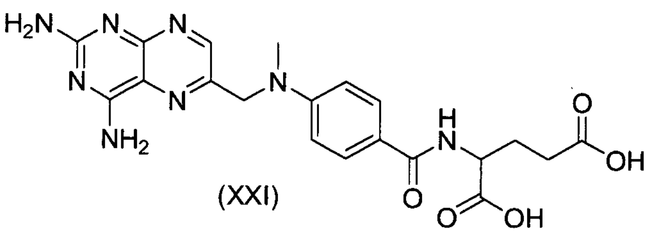

(XXI)

According to the present invention, Methotrexate is available by purchasing Methotrexate® or Rheumatrex® from Wyeth-Takeda.

(4) Microtubule inhibitors

A microtubule inhibitor of the invention refers to a substance having an effect of inhibiting the microtubule functions such as spindle forming function upon cell division and transporting function. For example, a microtubule inhibitor shows an anti-tumor effect by acting on microtubules in cells associated with highly active cell division, nerve cells or the like.

Examples of the microtubule inhibitor of the invention include Paclitaxel and Docetaxel, preferably Paclitaxel.
The microtubule inhibitor may be produced by a known method or may be purchased.
According to the present invention, Paclitaxel refers to (-)-(1S, 2R, 3S, 4S, 5R, 7S, 8S, 10R, 13S)-4,10-diacetoxy-2-benzoyloxy-5,20-epoxy,1,7-dihydroxy-9-oxotax-11-en-13-yl (2S, 3S)-3-benzoylamino-2-hydroxy-3-phenylpropionate and is a compound represented by Formula (XXII).

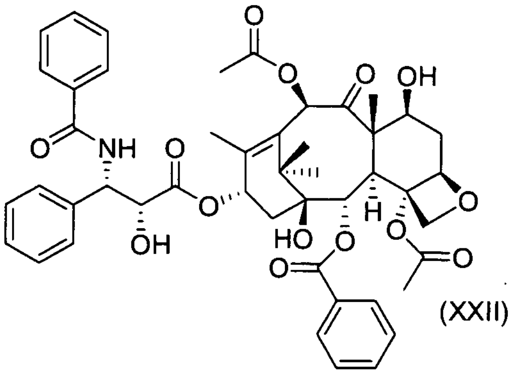

(XXII)

Paclitaxel is available by purchasing Taxol from Bristol.
Docetaxel is available by purchasing Taxotere® (Sanofi Aventis).

(5) Antibiotics

Preferably, an antibiotic of the present invention is an anti-tumoral antibiotic. An anti-tumoral antibiotic shows an anti-tumor effect by effecting DNA synthesis inhibition, DNA strand breakage or the like in a tumor cell.
According to the present invention, examples of an antibiotic include Doxorubicin (Adriamycin), Daunorubicin, Pirarubicin, Epirubicin, Idarubicin, Aclarubicin, Amrubicin, Mitoxantrone, preferably Doxorubicin.
An antibiotic may be produced by a known method or may be purchased.
According to the present invention, Doxorubicin refers to 10-[(3-amino-2,3,6-trideoxy-α-L-lyxo-hexopyranosyl) oxy]-7,8,9,10-tetrahydro-6,8,11-tri hydroxy-8-(hydroxyacetyl)-1-methoxy-(8S-cis)-5,12-naphthacenedione, and is a compound represented by Formula (XXIII).

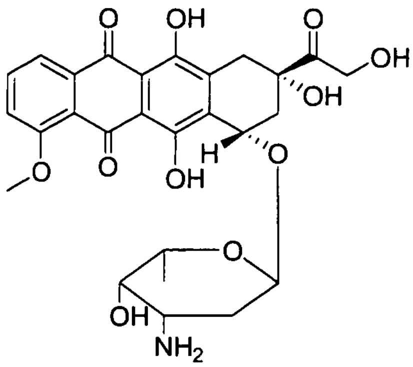

(XXIII)

Doxorubicin is available by purchasing Adriacin® (Kyowa Hakko).
Daunorubicin is available by purchasing Daunomycin® (Meiji Seika).
Piparubicin is available by purchasing Therarubicin® (Meiji Seika) or Pinorubin® (Mercian-Nippon Kayaku).
Epirubicin is available by purchasing Farmorubicin® (Pfizer-Kyowa Hakko).
Idarubicin is available by purchasing Idamycin® (Pfizer).
Aclarubicin is available by purchasing Aclacinon® (Mercian-Yamanouchi).
Amurubicin is available by purchasing Calsed® (Sumitomo Pharma).
Mitoxantrone is available by purchasing Novantron® (Wyeth-Takeda).

(6) Salts and solvates

A platinum complex, a DNA-topoisomerase I inhibitor, an antimetabolite, a microtubule inhibitor and an antibiotic may form pharmacologically acceptable salts with acids or bases. The compounds in (1) to (5) mentioned above may form pharmacologically acceptable salts that are different from those exemplified in (1) to (5) above. The above substances of the invention also comprise their pharmacologically acceptable salts. Examples of salts formed with acids include inorganic acid salts such as hydrochloride salts, hydrobromide salts, sulfate salts and phosphate salts, and salts formed with organic acids such as formic acid, acetic acid, lactic acid, succinic acid, fumaric acid, maleic acid, citric acid, tartaric acid, benzoic acid, methanesulfonic acid, benzenesulfonic acid, p-toluenesulfonic acid and trifluoroacetic acid. Examples of salts formed with bases include alkali metal salts such as sodium salt and potassium salt, alkaline earth metal salts such as calcium salt and magnesium salt, and salts formed with organic bases such as trimethylamine, triethylamine, pyridine, picoline, dicyclohexylamine and N,N'-dibenzylethylenediamine, arginine, lysine (organic amine salts), and ammonium salts.

The platinum complex, the DNA-topoisomerase I inhibitor, the antimetabolite, the microtubule inhibitor and the antibiotic may be an anhydride or may form a solvate such as a hydrate. The solvate may be either a hydrate or a nonhydrate, preferably a hydrate. The solvent may use water, alcohol (e.g., methanol, ethanol or n-propanol), dimethylformamide or the like.

If solvates and/or enantiomers of the above substances exist, these solvates and/or enantiomers are also comprised in the above substances of the invention. The substances of the invention also comprise at least one selected from a platinum complex, a DNA-topoisomerase I inhibitor, an antimetabolite, a microtubule inhibitor and an antibiotic that undergo metabolism *in vivo* such as oxidation, reduction, hydrolysis and conjugation. Moreover, the above substances of the invention also comprise substances that generate at least one selected from a platinum complex, a DNA-topoisomerase I inhibitor, an antimetabolite, a microtubule inhibitor and an antibiotic by undergoing metabolism *in vivo* such as oxidation, reduction and hydrolysis.

3. Pharmaceutical composition, kit, method for treating cancer and method for inhibiting angiogenesis

The present invention relates to a pharmaceutical composition, a kit, a method for treating cancer and a method for inhibiting angiogenesis, characterized by comprising a sulfonamide compound in combination with at least one substance selected from (i) a platinum complex, (ii) a DNA-topoisomerase I inhibitor, (iii) an antimetabolite, (iv) a microtubule inhibitor and (v) an antibiotic.

According to the present invention, a sulfonamide compound is as described in "1. Sulfonamide compound". For example, the sulfonamide compound is at least one compound selected from: (A) E7820 (Formula (V)); (B) a compound represented by General Formula (I), preferably LY186641 or LY295501; (C) a compound represented by General Formula (II), preferably LY-ASAP; (D) LY573636 (Formula (III)) and (E) CQS (Formula (IV)). More preferably, the sulfonamide compound is at least one compound selected from LY295501 and LY573636 and particularly preferably sodium salt of LY573636.

Alternatively, a sulfonamide compound of the present invention is preferably E7820.

According to the present invention, a platinum complex, a DNA-topoisomerase I inhibitor, an antimetabolite, a microtubule inhibitor and an antibiotic are as described in "2. Platinum complex, DNA-topoisomerase I inhibitor, Antimetabolite, Microtubule inhibitor and Antibiotic". (i) The platinum complex is preferably Oxaliplatin, (ii) the DNA-topoisomerase I inhibitor is preferably CPT-11, and (iii) the antimetabolite is preferably Gemcitabine. Alternatively, according to the present invention, (i) the platinum complex is preferably Cisplatin, (iii) the antimetabolite is preferably Methotrexate, (iv) the microtubule inhibitor is preferably Paclitaxel and (v) the antibiotic is preferably Doxorubicin.

According to the present invention, the sulfonamide compound and the substances of (i) to (v) above also comprise pharmacologically acceptable salts thereof, or solvates such as hydrates thereof.

The pharmaceutical composition of the invention comprising a sulfonamide compound in combination with at least one substance selected from (i) a platinum complex, (ii) a DNA-topoisomerase I inhibitor, (iii) an antimetabolite, (iv) a microtubule inhibitor and (v) an antibiotic. The pharmaceutical composition of the invention is useful for treating cancer and/or inhibiting angiogenesis.

According to the present invention, the term "in combination with" refers to a combination of compounds for combinational use, and includes both mode in which separate substances are administered in combination and as a mixture.

The pharmaceutical composition of the invention is also provided in another embodiment of a pharmaceutical composition comprising a sulfonamide compound, which is administered to a patient in combination with at least one substance selected from (i) a platinum complex, (ii) a DNA-topoisomerase I inhibitor, (iii) an antimetabolite, (iv) a microtubule inhibitor and (v) an antibiotic. The sulfonamide compound and at least one substance selected from (i) a platinum complex, (ii) a DNA-topoisomerase I inhibitor, (iii) an antimetabolite, (iv) a microtubule inhibitor and (v) an antibiotic may

be administered either simultaneously or separately. The term "simultaneous" refers to administrations at the same timing in a single administration schedule. In this case, it is not necessary to use completely the same hour and minute for administration. The term "separately" refers to administrations at different timings in a single administration schedule.

The kit of the invention comprises a set of a formulation comprising a sulfonamide compound and a formulation comprising at least one substance selected from the group consisting of (i) a platinum complex, (ii) a DNA-topoisomerase I inhibitor, (iii) an antimetabolite, (iv) a microtubule inhibitor and (v) an antibiotic. The formulations comprised in the kit of the invention are not limited to a particular form as long as they comprise a sulfonamide compound or at least one of the substances (i) to (v) above. The kit of the invention is useful for treating cancer and/or inhibiting angiogenesis.

In the kit of the invention, the formulation comprising a sulfonamide compound and the formulation comprising at least one of the substances (i) to (v) above may be mixed together or separately accommodated in a single package. They may be administered simultaneously or one may be administered preceding the other.

The pharmaceutical composition and/or the kit and the method for treating cancer and/or a method for inhibiting angiogenesis of the invention may be further combined with one or more additional anti-cancer drugs. The additional anti-cancer drugs are not particularly limited as long as they are formulations having an anti-tumor activity. Examples of the additional anti-cancer drug include 5-fluorouracil (5-FU), calcium folinate (Leucovorin), docetaxel (Taxotere®), gefitinib (Iressa®), erlotinib (Tarceva®, cetuximab (Erbitux®) and bevacizumab (Avastin®). Particularly preferable additional anti-cancer drugs are 5-fluorouracil, calcium folinate, gefitinib, erlotinib, cetuximab or bevacizumab when the type of cancer to be treated by the drug is colon cancer, and gefitinib, erlotinib, cetuximab or bevacizumab for pancreas cancer.

More examples of particularly preferable combinations of the compounds according to the invention are shown in Tables 1 and 2 for the cases of treating colon cancer and pancreas cancer by the therapeutic drug, respectively.

Table 1

| | Combined Compounds | | | | | |
|---|---|---|---|---|---|---|
| 1 | E7070 | Oxaliplatin | 5-FU | LV | Gefitinib | |
| 2 | E7820 | Oxaliplatin | 5-FU | LV | Gefitinib | |
| 3 | E7070 | Oxaliplatin | 5-FU | LV | Erlotinib | |
| 4 | E7820 | Oxaliplatin | 5-FU | LV | Erlotinib | |
| 5 | E7070 | Oxaliplatin | 5-FU | LV | Cetuximab | |
| 6 | E7820 | Oxaliplatin | 5-FU | LV | Cetuximab | |
| 7 | E7070 | Oxaliplatin | 5-FU | LV | Gefitinib | Bevacizumab |
| 8 | E7820 | Oxaliplatin | 5-FU | LV | Gefitinib | Bevacizumab |
| 9 | E7070 | Oxaliplatin | 5-FU | LV | Erlotinib | Bevacizumab |
| 10 | E7820 | Oxaliplatin | 5-FU | LV | Erlotinib | Bevacizumab |
| 11 | E7070 | Oxaliplatin | 5-FU | LV | Cetuximab | Bevacizumab |
| 12 | E7820 | Oxaliplatin | 5-FU | LV | Cetuximab | Bevacizumab |
| 13 | E7070 | CPT-11 | 5-FU | LV | Gefitinib | |
| 14 | E7820 | CPT-11 | 5-FU | LV | Gefitinib | |
| 15 | E7070 | CPT-11 | 5-FU | LV | Erlotinib | |
| 16 | E7820 | CPT-11 | 5-FU | LV | Erlotinib | |
| 17 | E7070 | CPT-11 | 5-FU | LV | Cetuximab | |
| 18 | E7820 | CPT-11 | 5-FU | LV | Cetuximab | |
| 19 | E7070 | CPT-11 | 5-FU | LV | Gefitinib | Bevacizumab |
| 20 | E7820 | CPT-11 | 5-FU | LV | Gefitinib | Bevacizumab |
| 21 | E7070 | CPT-11 | 5-FU | LV | Erlotinib | Bevacizumab |
| 22 | E7820 | CPT-11 | 5-FU | LV | Erlotinib | Bevacizumab |
| 23 | E7070 | CPT-11 | 5-FU | LV | Cetuximab | Bevacizumab |
| 24 | E7820 | CPT-11 | 5-FU | LV | Cetuximab | Bevacizumab |

Table 1 shows preferable combinations of the invention where the type of cancer to be treated by the therapeutic drug for cancer is colon cancer. In the table, LV represents calcium folinate.

Table 2

| | Combined Compounds | | | |
|---|---|---|---|---|
| 1 | E7070 | Gemcitabine | Gefitinib | |
| 2 | E7820 | Gemcitabine | Gefitinib | |
| 3 | E7070 | Gemcitabine | Erlotinib | |
| 4 | E7820 | Gemcitabine | Erlotinib | |
| 5 | E7070 | Gemcitabine | Cetuximab | |
| 6 | E7820 | Gemcitabine | Cetuximab | |
| 7 | E7070 | Gemcitabine | Gefitinib | Bevacizumab |
| 8 | E7820 | Gemcitabine | Gefitinib | Bevacizumab |
| 9 | E7070 | Gemcitabine | Erlotinib | Bevacizumab |
| 10 | E7820 | Gemcitabine | Erlotinib | Bevacizumab |
| 11 | E7070 | Gemcitabine | Cetuximab | Bevacizumab |
| 12 | E7820 | Gemcitabine | Cetuximab | Bevacizumab |

Table 2 shows preferable combinations of the invention where the type of cancer to be treated by the therapeutic drug for cancer is pancreas cancer.

The pharmaceutical composition and/or the kit of the invention can be used as a therapeutic drug for cancer or as an angiogenesis inhibitor.

Treatments according to the present invention comprise symptomatic relief of the disease, progression delay of symptoms of the disease, elimination of the symptoms of the disease, improvement of prognosis of the disease, and prevention of recurrence of the disease.

A therapeutic drug for cancer according to the invention comprises those that contain an anti-tumor agent, a drug for improving prognosis of cancer, a drug for preventing cancer recurrence, an antimetastatic drug or the like.

The effect of cancer treatment can be confirmed by observation of X-ray pictures, CT or the like, histopathologic diagnosis by biopsy, or tumor marker value.

The pharmaceutical composition and/or the kit of the invention can be administered to mammals (e.g., human, rat, rabbit, sheep, pig, cattle, cat, dog and monkey).

Examples of the types of cancers targeted by the therapeutic drug for cancer include but not limited to at least one selected from the group consisting of brain tumor, cervical cancer, esophageal cancer, tongue cancer, lung cancer, breast cancer, pancreas cancer, gastric cancer, small intestinal and duodenal cancer, colon cancer (colon cancer and rectal cancer), bladder cancer, renal cancer, liver cancer, prostate cancer, uterin cancer, ovarian cancer, thyroid grand cancer, gallbladder cancer, pharyngeal cancer, sarcoma (e.g., osteosarcoma, chondrosarcoma, Kaposi's sarcoma, myosarcoma, angiosarcoma, fibrosarcoma, etc.), leukemia (e.g., chronic myelocytic leukemia (CML), acute myelocytic leukemia (AML), chronic lymphocytic leukemia (CLL), acute lymphocytic leukemia (ALL), lymphoma, multiple myeloma (MM), etc.) and melanoma. Preferably, the type of cancer targeted by the therapeutic drug for cancer is at least one selected from the group consisting of colon cancer and pancreas cancer.

The pharmaceutical composition and/or the kit of the invention may be administered orally or parenterally.

When the pharmaceutical composition and/or kit of the invention is used, the given dose of the sulfonamide compound differs depending on the degree of the symptom, age, sex, weight and sensitivity difference of the patient, administration mode, administration period, administration interval, and nature, prescription and type of the pharmaceutical formulation and the type of the active ingredient. Usually, but without limitation, the dose of the sulfonamide compound is 10-6000 mg/day, preferably 50-4000 mg/day, more preferably 50-2000 mg/day for an adult (weight 60 Kg), which may be administered once to three times a day.

When using the pharmaceutical composition and/or the kit of the invention, the given dose of the at least one substance selected from the group consisting of (i) a platinum complex, preferably Oxaliplatin or Cisplatin, (ii) a DNA-topoisomerase I inhibitor, preferably CPT-11, (iii) an antimetabolite, preferably Gemcitabine or Methotrexate, (iv) a microtubule inhibitor, preferably Paclitaxel and (v) an antibiotic, preferably Doxorubicin, is usually, but not particularly limited to, 10-6000 mg/day, preferably 50-4000 mg/day, more preferably 50-2000 mg/day for an adult, which may be administered once to three times a day.

The amount of the sulfonamide compound used is not particularly limited, and differs depending on the individual combination with the at least one substance selected from the group consisting of (i) a platinum complex, preferably Oxaliplatin or Cisplatin, (ii) a DNA-topoisomerase I inhibitor, preferably CPT-11, (iii) an antimetabolite, preferably Gemcitabine or Methotrexate, (iv) a microtubule inhibitor, preferably Paclitaxel and (v) an antibiotic, preferably Doxorubicin. For example, the amount of the sulfonamide compound is about 0.01-100 times (weight ratio), more preferably about

0.1-10 times (weight ratio) of the amount of the at least one substance selected from (i) to (v).

The pharmaceutical composition of the invention may be made into various dosage forms, for example, into solid oral formulations or parenteral formulations such as injection, suppository, ointment and skin patch.

Furthermore, the sulfonamide compound and the at least one substance selected from the group consisting of (i) a platinum complex, (ii) a DNA-topoisomerase I inhibitor, (iii) an antimetabolite, (iv) a microtubule inhibitor and (v) an antibiotic contained in the kit of the invention may individually be made into various dosage forms, for example, into solid oral formulations or parenteral formulations such as injection, suppository, ointment and skin patch.

In order to prepare a solid oral formulation, an excipient, and if necessary, a binder, disintegrant, lubricant, colorant, a flavoring agent or the like may be added to a principal agent, and then made into a tablet, a coated tablet, granule, subtle granule, powder, a capsule or the like according to a conventional method. In addition, a non-solid oral formulation such as a syrup agent can also be prepared appropriately.

For example, lactose, cornstarch, sucrose, glucose, sorbit, crystalline cellulose, silicon dioxide or the like may be used as the excipient; for example, polyvinyl alcohol, ethyl cellulose, methyl cellulose, gum arabic, hydroxypropyl cellulose, hydroxypropylmethyl cellulose or the like may be used as the binder; for example, magnesium stearate, talc, silica or the like may be used as the lubricant; those that are allowed to be added to pharmaceutical preparations may be used as the colorant; and for example, cocoa powder, menthol, aromatic acid, peppermint oil, camphor, cinnamon powder or the like may be used as the flavoring agent. Of course, if necessary, these tablets and granule may be coated appropriately with sugar coating, gelatin coating or else.

When an injection is to be prepared, if necessary, the principal agent may be added with a pH adjuster, a buffer, a suspending agent, a solubilizing aid, a stabilizer, an isotonizing agent, a preservative or the like, and may be made into an intravenously, subcutaneously or intramuscularly injection or an intravenous dorip injection according to a conventional method. In this case, if necessary, it is also prepared a lyophilized form by a conventional technique.

Examples of the suspending agent may include methyl cellulose, Polysorbate 80, hydroxyethyl cellulose, gum arabic, powdered tragacanth, sodium carboxy methyl cellulose and polyoxyethylene sorbitan monolaurate.

Examples of the solubilizing aid may include polyoxyethylene hydrogenated castor oil, Polysorbate 80, nicotine acid amide, polyoxyethylene sorbitan monolaurate, macrogol, and ethyl ester of castor oil fatty acid.

Examples of the stabilizer may include sodium sulfite and sodium metasulfite; examples of the preservative may include methyl parahydroxybenzoate, ethyl parahydroxybenzoate, sorbic acid, phenol, cresol and chlorocresol.

Besides the sulfonamide compound and the at least one substance selected from the group consisting of (i) a platinum complex, (ii) a DNA-topoisomerase I inhibitor, (iii) an antimetabolite, (iv) a microtubule inhibitor and (v) an antibiotic, the pharmaceutical composition and/or the kit of the invention can also comprise a packaging container, an instruction, a package insert or the like. The packaging container, the instruction, the package insert or the like may include description of combinations for combinational use of the compound, and usage and dosage in the case of administering separate substances in combination or in the case of administering them in the form of a mixture. The usage and dosage may be described referring to the related description above.

The kit of the invention may also comprise: (a) at least one selected from the group consisting of a packaging container, an instruction and a package insert describing combinational use of the sulfonamide compound and the at least one substance selected from the group comprising of (i) a platinum complex, (ii) a DNA-topoisomerase I inhibitor, (iii) an antimetabolite, (iv) a microtubule inhibitor and (v) an antibiotic; and (b) a pharmaceutical composition comprising the sulfonamide compound. The kit is useful for treating cancer and/or for inhibiting angiogenesis. The pharmaceutical composition comprising the sulfonamide compound is useful for treating cancer and/or for inhibiting angiogenesis. The packaging container, the instruction, the package insert or the like may include the description of combinations for combinational use of the sulfonamide compound and the at least one substance selected from (i) to (v) above, and usage and dosage for combinational use in the case of administering separate substances in combination or in the case of administering them in the form of a mixture. The usage and dosage may be determined by referring to the description of Pharmaceutical composition/Kit above.

The present invention also comprises use of a sulfonamide compound for producing a pharmaceutical composition in combination with at least one substance selected from the group consisting of (i) a platinum complex, (ii) a DNA-topoisomerase I inhibitor, (iii) an antimetabolite, (iv) a microtubule inhibitor and (v) an antibiotic. According to the use of the invention, the pharmaceutical composition is useful for treating cancer and/or for inhibiting angiogenesis.

The present invention also comprises a method for treating cancer and/or a method for inhibiting angiogenesis comprising simultaneously or separately administering a sulfonamide compound and at least one substance selected from the group consisting of (i) a platinum complex, (ii) a DNA-topoisomerase I inhibitor, (iii) an antimetabolite, (iv) a microtubule inhibitor and (v) an antibiotic to a patient. According to the method of the invention for treating cancer and/or inhibiting angiogenesis, the route and the method for administering the sulfonamide compound and the at least one substance selected from (i) to (v) above are not particularly limited but reference may be made to the description of the pharmaceutical composition of the invention above.

Hereinafter, the present invention will be described by way of specific examples, although the present invention

is not limited thereto.

EXAMPLE 1: Effects of combinational use of E7820 and anti-cancer drugs Paclitaxel, SN38 (active form of CPT-11), Methotrexate, Cisplatin, Gemcitabine or Doxorubicin on cell proliferation in vascular endothelial cell proliferation assay *(in vitro)*

Human umbilical vein endothelial cells were suspended in bullet kit EGM-2 (Cambrex) as a culture medium to 1 x $10^4$ cells/ml, and 100 μl each of this solution was added to each well of a 96 well plate for cultivation in a 5% carbon dioxide incubator at 37°C. On the following day, a solution containing E7820, a solution containing an anti-cancer drug for combinational use and a solution containing both compounds, i.e., E7820 and the anti-cancer drug, were each diluted in the culture medium. These diluted solutions were added to the cultured wells for 100 μl/well for further cultivation.

Three days later, 10 μl of Cell Counting Kit-8 solution (Cell Counting Kit-8, Wako Pure Chemical Industries) was added, cultured for 2-3 hours at 37°C, and absorbance at 450 nm was determined with a plate reader (Corona Electric Co., Ltd.). The effect of the combinational use was calculated according to the formula of Chou et al (Adv. Enzyme Regul., 22, 27-55, 1984).

As a result, the combination of E7820 and Paclitaxel, SN38 (active form of CPT-11), Methotrexate, Cisplatin, Gemcitabine or Doxorubicin showed stronger antiproliferative effect than that obtained with E7820, Paclitaxel, SN38 (active form of CPT-11), Methotrexate, Cisplatin, Gemcitabine or Doxorubicin alone. Since the combination index (CI) obtained with combinational use of E7820 and Paclitaxel, SN38 (active form of CPT-11), Methotrexate, Cisplatin, Gemcitabine or Doxorubicin was 1 or less, E7820 was found to indicate a synergistic antiproliferative effect by combinational use with Paclitaxel, SN38 (active form of CPT-11), Methotrexate, Cisplatin, Gemcitabine or Doxorubicin (Table 3). This effect was remarkable as compared to an effect observed with general combinational use, which was unpredictable by those skilled in the art.

Table 3

| Combined subject | Fractional inhibition (fa) | Combination index (CI) | Combined effect |
|---|---|---|---|
| Paclitaxel | 0.7 | 0.8 | Synergistic |
| | 0.8 | 0.7 | Synergistic |
| | 0.9 | 0.6 | Synergistic |
| | 0.95 | 0.7 | Synergistic |
| SN38 | 0.6 | 0.8 | Synergistic |
| | 0.7 | 0.6 | Synergistic |
| | 0.8 | 0.5 | Synergistic |
| | 0.9 | 0.5 | Synergistic |
| | 0.95 | 0.6 | Synergistic |
| Methotrexate | 0.9 | 0.9 | Synergistic |
| | 0.95 | 0.8 | Synergistic |
| Cisplatin | 0.6 | 0.9 | Synergistic |
| | 0.7 | 0.7 | Synergistic |
| | 0.8 | 0.5 | Synergistic |
| | 0.9 | 0.4 | Synergistic |
| | 0.95 | 0.3 | Synergistic |
| Gemcitabine | 0. 8 | 0.9 | Synergistic |
| | 0.9 | 0.9 | Synergistic |
| Doxorubicin | 0.6 | 0.9 | Synergistic |
| | 0.7 | 0.8 | Synergistic |
| | 0.8 | 0.6 | Synergistic |
| | 0.9 | 0.5 | Synergistic |
| | 0.95 | 0.4 | Synergistic |

Table 3 shows the synergistic antiproliferative effect by E7820 and anti-cancer drugs in vascular endothelial cell proliferation assay *(in vitro).*

EXAMPLE 2: Effects of combinational use of E7820 and anti-cancer drugs Paclitaxel, SN38 (active form of CPT-11), Cisplatin, Gemcitabine or Doxorubicin on lumen formation in vascular endothelial cell lumen formulation assay *(in vitro)*

Four-hundred μl of type I collagen gel (Nitta Gelatin) was dispensed in a 24-well plate (FALCON) and left in a $CO_2$ incubator at 37°C for 40 minute for gelatinization. A serum-free medium (Human endothelial-SFM Basal Growth Medium, Invitrogen) containing 20 ng/ml EGF (GIBCO BRL) was prepared. Two-hundred μl of this solution was dispensed in the wells containing type I collagen gel. Human umbilical vein endothelial cell (HUVEC) line was suspended in a serum-free medium (Human endothelial-SFM Basal Growth Medium, GIBCO BRL) to prepare 5 x $10^5$ cells/ml cell suspension. Two-hundred μl of this suspension was seeded on the wells containing the type I collagen gel and the serum-free medium and cultured overnight.

On the following day, the wells were overlaid with 400 μl of type I collagen gel and left in a $CO_2$ incubator at 37°C for 3 hours for gelatinization. Then, the serum-free media (containing 10 ng/ml EGF and 20 ng/ml VEGF (Genzyme Techne Corp.)) containing E7820 alone, containing an anti-cancer drug alone and containing both E7820 and the anti-cancer drug for combinational use were added for 1.5 ml each and further cultured in a $CO_2$ incubator at 37°C for 3 days.

After the cultivation, 400 μl of 3.3 mg/ml MTT (SIGMA) solution was dispensed in the wells and reacted in a $CO_2$ incubator at 37°C for 3 hours for the cells to develop color. Images of lumens formed by HUVEC were captured (M-3204C, OLYMPUS) under a microscope (SZX12, OLYMPUS). The captured images were subjected to image analysis software Mac SCOPE 2.56 (MITANI) to determine the lengths of the lumens to quantify lumen formulation by HUVEC. The effect of the combinational use was calculated according to the formula of Chou et al (Adv. Enzyme Regul., 22, 27-55, 1984).

As a result, the combination of E7820 and Paclitaxel, SN38 (active form of CPT-11), Cisplatin, Gemcitabine or Doxorubicin showed stronger lumen formation inhibitory effect than that obtained with E7820, Paclitaxel, SN38 (active form of CPT-11), Cisplatin, Gemcitabine or Doxorubicin alone. Since the combination index (CI) obtained with combinational use of E7820 and Paclitaxel, SN38 (active form of CPT-11), Cisplatin, Gemcitabine or Doxorubicin was 1 or less, E7820 was found to indicate a synergistic lumen formation inhibitory effect by combinational use with Paclitaxel, SN38 (active form of CPT-11), Cisplatin, Gemcitabine or Doxorubicin (Table 4). This effect was remarkable as compared to an effect observed with general combinational use, which was unpredictable by those skilled in the art.

Table 4

| Combined subject | Fractional inhibition (fa) | Combination index (CI) | Combined effect |
|---|---|---|---|
| Paclitaxel | 0.7 | 0.9 | Synergistic |
| | 0.8 | 0.7 | Synergistic |
| | 0.9 | 0.6 | Synergistic |
| | 0.95 | 0.5 | Synergistic |
| SN38 | 0.3 | 0.7 | Synergistic |
| | 0.4 | 0.6 | Synergistic |
| | 0.5 | 0.5 | Synergistic |
| | 0.6 | 0.4 | Synergistic |
| | 0.7 | 0.4 | Synergistic |
| | 0.8 | 0.3 | Synergistic |
| | 0.9 | 0.2 | Synergistic |
| | 0.95 | 0.2 | Synergistic |
| Cisplatin | 0.05 | 0.7 | Synergistic |
| | 0.1 | 0.6 | Synergistic |
| | 0.2 | 0.6 | Synergistic |
| | 0.3 | 0.6 | Synergistic |
| | 0.4 | 0.5 | Synergistic |
| | 0.5 | 0.5 | Synergistic |
| | 0.6 | 0.5 | Synergistic |
| | 0.7 | 0.5 | Synergistic |
| | 0.8 | 0.5 | Synergistic |
| | 0.9 | 0.5 | Synergistic |
| | 0.95 | 0.4 | Synergistic |

(continued)

| Combined subject | Fractional inhibition (fa) | Combination index (CI) | Combined effect |
|---|---|---|---|
| Gemcitabine | 0.3 | 0.7 | Synergistic |
| | 0.4 | 0.4 | Synergistic |
| | 0.5 | 0.3 | Synergistic |
| | 0.6 | 0.3 | Synergistic |
| | 0.7 | 0.2 | Synergistic |
| | 0.8 | 0.2 | Synergistic |
| | 0.9 | 0.3 | Synergistic |
| | 0.95 | 0.3 | Synergistic |
| Doxorubicin | 0.2 | 0.6 | Synergistic |
| | 0.3 | 0.4 | Synergistic |
| | 0.4 | 0.3 | Synergistic |
| | 0.5 | 0.3 | Synergistic |
| | 0.6 | 0.2 | Synergistic |
| | 0.7 | 0.2 | Synergistic |
| | 0.8 | 0.1 | Synergistic |
| | 0.9 | 0.1 | Synergistic |
| | 0.95 | 0.1 | Synergistic |

Table 4 shows the synergistic effect of E7820 and anti-cancer drugs on lumen formation inhibition in vascular endothelial cells.

From these results, by combining E7820 with at least one substance selected from the group consisting of a platinum complex, a DNA-topoisomerase I inhibitor, an antimetabolite, a microtubule inhibitor and an antibiotic, a pharmaceutical composition and a kit showing a remarkable angiogenesis inhibition activity and a method for inhibiting angiogenesis are provided. Thus, the pharmaceutical composition, the kit and the method of the invention can be used for treating cancer and for inhibiting angiogenesis.

EXAMPLE 3: Combinational use of E7820 and Oxaliplatin in subcutaneous transplant model of human colon cancer cell line (Colo320DM)

Human colon cancer cell line Colo320DM (obtained from Dainippon Pharmaceutical) was cultured in RPMI1640 (containing 10% FBS) in a 5% carbon dioxide incubator at 37°C to about 80% confluence, and the cells were collected with trypsin-EDTA. Using a phosphate buffer containing 50% matrigel, 7.5 x $10^7$ cells/mL suspension was prepared, and 0.1 mL each of the resulting cell suspension was subcutaneously transplanted to a nude mouse at the side of its body. Six days after the transplantation, administrations of E7820 (50 mg/kg, twice a day, for two weeks, oral administration) and Oxaliplatin (Eloxatino®, Sanofi Aventis) (10 mg/kg, once in four days, total of three times, intravenous administration) alone or in combination were initiated. The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{Minor axis of tumor})^2 (\text{mm}^2)/2$$

$$\text{Relative Tumor Volume RTV} = \text{Tumor volume on measurement day/Tumor volume on the first administration day}$$

If statistically significant interaction was observed in the combinational use group by two-way ANOVA, a synergistic effect was considered to exist between E7820 and Oxaliplatin.

As a result, E7820 was found to produce a synergistic effect when used in combination with Oxaliplatin, and their combinational use showed a superior anti-tumor effect as copared with the effect obtained with E7820 or Oxaliplatin alone (Table 5 and Figure 1). In addition, combinational use of E7820 and Oxaliplatin also showed a remarkable anti-tumor effect that cannot be seen with Oxaliplatin alone (Table 5 and Figure 1).

Table 5

| Administered subject | Relative tumor volume on Day 15 Average ± standard deviation | Two-way ANOVA |
|---|---|---|
| Control (untreated) | 11.99 ± 1.35 | |
| E7820 50 mg/kg | 7.05 ± 1.15 | |
| Oxaliplatin 10 mg/kg | 11.7 ± 1.46 | |
| E7820 50 mg/kg + Oxaliplatin 10 mg/kg | 3.87 ± 1.53 | p<0.05 Synergistic effect |

Table 5 shows anti-tumor effects obtained by the use of E7820 alone, the use of Oxaliplatin alone and the combinational use of E7820 and Oxaliplatin in Colo320DM nude mouse subcutaneous transplant models. The first day of administration was considered Day 1.

From the obtained results, the combination of E7820 and Oxaliplatin provides a pharmaceutical composition and a kit that show a remarkable anti-tumor activity, and a method for treating cancer, and thus the pharmaceutical composition, the kit and the method of the invention can be used for treating cancer.

EXAMPLE 4: Combinational use of E7820 and CPT-11 in subcutaneous transplant model *(in vivo)* of human colon cancer cell line (Colo320DM)

Human colon cancer cell line Colo320DM (purchased from Dainippon Pharmaceutical) was cultured in RPMI1640 (containing 10% FBS) in a 5% carbon dioxide incubator at 37°C to about 80% confluence, and the cells were collected with trypsin-EDTA. Using a phosphate buffer containing 50% matrigel, 7.5 x $10^7$ cells/mL suspension was prepared, and 0.1 mL each of the resulting cell suspension was subcutaneously transplanted to a nude mouse at the side of its body. Six days after the transplantation, administrations of E7820 (50 mg/kg, twice a day, for two weeks, oral administration) and CPT-11 (Topotecin®, Daiichi Pharm) (100 mg/kg, once in four days, total of three times, intravenous administration) alone or in combination were initiated. The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{minor axis of tumor})^2 (\text{mm}^2)/2$$

$$\text{Relative Tumor Volume RTV} = \text{Tumor volume on measurement day/Tumor volume on the first administration day}$$

If statistically significant interaction was observed in the combinational use group by two-way ANOVA, a synergistic effect is considered to exist between E7820 and CPT-11.

As a result, E7820 was found to produce a synergistic effect when used in combination with CPT-11, and their combinationl use showed a superior anti-tumor effect as compared with the effect obtained with E7820 or CPT-11 alone (Table 6 and Figure 2). In addition, combinational use of E7820 and CPT-11 also showed a remarkable anti-tumor effect that cannot be seen with CPT-11 alone (Table 6 and Figure 2).

Table 6

| Administered subject | Relative tumor volume on Day 15 Average ± standard deviation | Two-way ANOVA |
|---|---|---|
| Control (untreated) | 11.99 ± 1.35 | |
| E7820 50 mg/kg | 7.05 ± 1.15 | |
| CPT-11 100 mg/kg | 1.79 ± 0.41 | |
| E782050mg/kg E7820 50 mg/kg + CPT-11 100 mg/kg | 0.24 ± 0.03 | $p < 0.01$ Synergistic effect |

Table 6 shows anti-tumor effects obtained by the use of E7820 alone, the use of CPT-11 alone and the combinational use of E7820 and CPT-11 in Colo320DM nude mouse subcutaneous transplant models. The first day of administration was considered Day 1.

From the obtained results, the combination of E7820 and CPT-11 provides a pharmaceutical composition and a kit that show a remarkable anti-tumor activity, and a method for treating cancer, and thus the pharmaceutical composition, the kit and the method of the invention can be used for treating cancer.

EXAMPLE 5: Combinational use of E7820 and Gemcitabine in subcutaneous transplant model *(in vivo)* of human pancreas cancer cell line (KP-1)

Human pancreas cancer cell line KP-1 (obtained from the National Kyushu Cancer Center) was cultured in RPMI1640 (containing 10% FBS) in a 5% carbon dioxide incubator at 37°C to about 80% confluence, and the cells were collected with trypsin-EDTA. Using a phosphate buffer, 1 x $10^8$ cells/mL suspension was prepared, and 0.1 mL each of the resulting cell suspension was subcutaneously transplanted to a nude mouse at the side of its body. Eleven days after the transplantation, administrations of E7820 (50 mg/kg, twice a day, for three weeks, oral administration) and Gemcitabine (GEMZAR®, Eli Lilly Japan) (200 mg/kg, once in three days, total of four times, intravenous administration) alone or in combination were initiated. The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{Minor axis of tumor})^2 (\text{mm}^2)/2$$

$$\text{Relative Tumor Volume RTV} = \text{Tumor volume on measurement day/Tumor volume on the first administration day}$$

If statistically significant interaction was observed in the combinational use group by two-way ANOVA, a synergistic effect was considered to exist between E7820 and Gemcitabine.

As a result, E7820 was found to produce a synergistic effect when used in combination with Gemcitabine, and their combinational use showed a superior anti-tumor effect as compared with the effect obtained with E7820 or Gemcitabine alone (Table 7 and Figure 3). In addition, combinational use of E7820 and Gemcitabine also showed a remarkable anti-tumor effect that cannot be seen with Gemcitabine alone (Table 7 and Figure 3).

Table 7

| Administered subject | Relative tumor volume on Day 22 Average ± standard deviation | Two-way ANOVA |
|---|---|---|
| Control (untreated) | 14.8 ± 2.28 | |
| E7820 50 mg/kg | 8.81 ± 2.57 | |
| Gemcitabine 200 mg/kg | 4.74 ± 1.50 | |

(continued)

| Administered subject | Relative tumor volume on Day 22 Average ± standard deviation | Two-way ANOVA |
|---|---|---|
| E7820 50 mg/kg + Gemcitabine 200 mg/kg | 0.96 ± 0.31 | $p < 0.01$ Synergistic effect |

Table 7 shows anti-tumor effects obtained by the use of E7820 alone, the use of Gemcitabine alone and the combinational use of E7820 and Gemcitabine in KP-1 nude mouse subcutaneous transplant models. The first day of administration was considered Day 1.

From the obtained results, the combination of E7820 and Gemcitabine provides a pharmaceutical composition and a kit that show a remarkable anti-tumor activity, and a method for treating cancer, and thus the pharmaceutical composition, the kit and the method of the invention can be used for treating cancer.

EXAMPLE 6: Quantification of DNA-topoisomerase II mDNA

2 x $10^6$ of human umbilical vein endothelial cells were seeded in a 25 $cm^2$ cell culture bottle and cultured in a $CO_2$ incubator at 37°C using a EGM-2 medium (Sanko Junyaku) overnight. On the following day, E7820 was added to give 1 μM. Six hours later, RNA was prepared from the cells. Specifically, the medium was removed from the cells. The cells were added with and dissolved in 3 ml of ISOGEN (Wako Pure Chemical Industries), added with an equal volume of $CHC1_3$ and agitated. Then, the water phase was extracted. Half the amount of the water phase of isopropanol was added, left to stand for 5 minutes and centrifuged to collect the precipitate. The precipitate was washed with 70% ethanol, added with and dissolved in sterile water and absorbance at 260 nm was determined with a spectrophotometer to quantify the RNA amount.

Subsequently, TaqMan Gold RT-PCR kit (Applied Biosystems) was used to perform RT-PCR reaction. Specifically, 0.1 μg of RNA was added to 50 μl of the reaction solution, and subjected to reactions at 25°C for 10 minutes, 48°C for 30 minutes and 95°C for 5 minutes to prepare cDNA. Then, a primer for determining DNA-topoisomerase II mRNA (ABI Taqman probe Hs00172214ml) was used for PCR reaction and then ABI7700 (Applied Biosystems) was used for quantifying RNA amount.

As a result, the amount of DNA-topoisomerase II mRNA was 4.4 μg/ml for the untreated group and 1.6 μg/ml for the E7820-treated group.

DNA-topoisomerase I inhibition by an anti-cancer drug has been reported to increase DNA-topoisomerase II in the tumor (Kim R, Hirabayashi N, Nishiyama M, et al. Experimental studies on biochemical modulation targeting topoisomerase I and II in human tumor xenografts in nude mice. Int J Cancer. 1992; 50: 760-6., Whitacre CM, Zborowska E, Gordon NH, et al. Topotecan increases topoisomerase II alpha levels and sensitivity to treatment with etoposide in schedule-dependent process. Cancer Res. 1997; 57: 1425-8). Accordingly, E7820 seems to show a synergistic anti-tumor effect in combination with a DNA-topoisomerase I inhibitor based on inhibition of the expression of DNA-topoisomerase II.

Thus, a sulfonamide compound was strongly suggested to show a synergistic anti-tumor effect when combined with not only CPT-11 but also when combined with other DNA-topoisomerase I inhibitors.

EXAMPLE 7: DNA microarray analysis

(1) Cell culture, compound treatment and RNA extraction

For the purpose of examining changes in the gene expression induced by the compounds by DNA microarray analysis, human colon cancer-derived cell line HCT116 (American Type Culture Collection, Manassas, VA, U.S.A.) and human leukemia-derived cell line MOLT-4 (American Type Culture Collection, Manassas, VA, U.S.A.) were cultured in RPMI-1640 media supplemented with 10% fetal bovine serum, 100 units/ml penicillin and 100 μg/ml streptomycin. The following cultivation and compound treatment took place in an incubator set to 5% $CO_2$ and 37°C. The HCT116 cells and the MOLT-4 cells were seeded on 10 cm-diameter cell culture dishes at 2.0 x $10^6$ cells/dish, cultured for 24 hours and subjected to the following compound treatments.

For the HCT116 cells, 12 compounds, i.e., E7820 (0.8 μM), E7070 (0.8 μM), LY295501 (30 μM), CQS (8 μM), adriamycin (0.2 μM), daunomycin (0.2 μM), ICRF154 (80 μM), ICRF159 (80 μM), kenpaullone (10 μM), alsterpullone (10 μM), trichostatin A (0.1 μM) and rapamycin (80 μM) were assessed. On the other hand, for the MOLT-4 cells, E7070 (0.8 μM) was assessed. Herein, adriamycin and daunomycin are compounds known as DNA intercalative DNA topoisomerase II inhibitors, ICRF154 and ICRF159 are compounds known as catalytic DNA topoisomerase II inhibitors, kenpaullone and alsterpullone are compounds known as cyclin-dependent kinase (CDK) inhibitors, trichostatin A is a

compound known as a histone deacetylase inhibitor and rapamycin is a compound known as an mTOR/FRAP inhibitor. The concentration of each compound used for the treatment was set to three to five-fold the 50% growth inhibitory concentration of each compound to the HCT116 cells (based on three days of antiproliferative activity using WST-8). The cells were collected 24 hours after the treatment at the concentration indicated in parentheses following each compound name above. Similarly, cells cultured for 24 hours without the addition of any compound were also collected.

Extraction of total RNA from the collected cells was performed using TRIZOL reagent (Invitrogen) according to the attached instruction.

(2) Analysis of gene expression using DNA microarray

The resulting RNA was dissolved in 100 μl of diethylpyrocarbonate (DEPC)-treated sterilized water, purified using an RNeasy column (QIAGEN), and double-stranded cDNA was synthesized using SuperScript Choice System (Invitrogen) and T7-d$(T)_{24}$ primers.

First, to 10 μg RNA, 5 μM T7-d$(T)_{24}$primer, 1x First strand buffer, 10 mM DTT, 500 μM dNTP mix and 20 units/μl SuperScript II Reverse Transcriptase were added and reacted at 42°C for an hour to synthesize single-stranded DNA. Subsequently, 1x Second strand buffer, 200 μM dNTP mix, 67 U/ml DNA ligase, 270 U/ml DNA polymerase I and 13 U/ml RNase H were added and reacted at 16°C for two hours to synthesize double-stranded cDNA. Furthermore, 67 U/ml T4 DNA polymerase I was added, reacted at 16°C for 5 minutes and then 10 μl of 0.5 M EDTA was added to terminate the reaction.

The obtained cDNA was purified with phenol/chloroform, and subjected to labeling reaction with biotinylated UTP and CTP using RNA Transcript Labeling Kit (Enzo Diagnostics) according to the attached instruction. The reaction product was purified using an RNeasy column, heated in 200 mM Tris acetic acid (pH8.1), 150 mM magnesium acetate and 50 mM potassium acetate at 94°C for 35 minutes for fragmentation of the cRNA.

The fragmented cRNA was hybridized to GeneChip (Affymetrix) Human Focus array in 100 mM MES, 1 M sodium salt, 20 mM EDTA and 0.01% Tween 20 at 45°C for 16 hours. After the hybridization, GeneChip was washed and stained according to protocol Midi_euk2 attached to the Affymetrix fluidics station. For staining, streptavidin-phycoerythrin and biotinylated anti-streptavidin goat antibody were used. The stained GeneChip was scanned using HP confocal microscope with argon ion laser (Hewlett Packard) to determine fluorescence intensity. Measurement took place at excitation and emission wavelengths of 488 nm and 570 nm, respectively.

All of the quantitative data analyses were carried out using GeneChip software (Affymetrix) and Gene Spring (Silicongenetics). GeneChip software was used for assessing changes in the gene expression induced by each compound, where gene expression was judged to have significantly "increased" or "decreased" when the quantified values in the two conditions, i.e., between the compound-treated group and the untreated group, were twice as different. Gene Spring was used for assessing the similarity of changes in gene expression induced by each compound, where hierarchical cluster analysis was conducted based on changes in the expressions of all genes on the Human Focus Array.

The results from the hierarchical cluster analysis for the HCT116 cells are shown in Figure 4.

As a result of the analysis, adriamycin and daunomycin, ICRF154 and ICRF159, and Kenpaullone and alsterpullone, each pair having the same action mechanism, gave similar genetic alterations (Figure 4). Thus, compounds having the same action mechanism were confirmed to give similar genetic alterations.

E7070, E7820, LY295501 and CQS gave similar genetic alterations (Figure 4). Therefore, E7070, E7820, LY295501 and CQS were considered to have the same or similar action mechanisms according to this analysis, suggesting that they give the same or similar genetic alterations and effects.

EXAMPLE 8: DNA microarray analysis

HCT116 cells were cultured in an RPMI-1640 medium supplemented with 10% fetal bovine serum, 100 units/ml penicillin and 100 μg/ml streptomycin. The following cultivation and compound treatment were carried out in an incubator at 5% $CO_2$ and 37°C. HCT116 cells were seeded in 10 cm-diameter cell culture dishes at 2.0 x $10^6$ cells/dish, cultured for 24 hours and subjected to the following compound treatment.

In this example, changes in the gene expression of HCT116 cells upon treatments with 12 compounds, i.e., E7820 (0.16 μM), E7070 (0.26 μM), LY186641 (59 μM), LY295501 (24 μM), LY-573636 (9.6 μM), CQS (4.0 μM), MST16 (100 μM), etoposide (3.6 μM), ethoxzolamide (410 μM), capsaicin (280 μM), trichostatin A (0.16 μM) and kenpaullone (7.1 μM) were examined.

MST16 is a compound known as a catalytic DNA topoisomerase II inhibitor, etoposide is a compound known as a DNA topoisomerase II inhibitor that induces formation of a cleavable complex, ethoxzolamide is a compound known as a carbonic anhydrase inhibitor, capsaicin is a compound known as a tumor-specific plasma membrane NADH oxidase inhibitor, trichostatin A is a compound known as a histone deacetylase inhibitor and kenpaullone is a compound known as a cyclin-dependent kinase (CDK) inhibitor.

The concentration of each compound used for the treatment was set to twice the 50% growth inhibitory concentration of each compound to the HCT116 cells (based on three days of antiproliferatrive actvity using MTT). The cells were collected 24 hours after the treatment at the concentration indicated in parentheses following each compound name above. Similarly, cells cultured for 24 hours without the addition of any compound were also collected.

Total RNA extraction from the collected cells was performed using TRIZOL reagent (Invitrogen) according to the attached instruction.

Gene expression analysis using a DNA microarray was carried out in the same manner as "(2) Analysis of gene expression using DNA microarray" in Example 7.

This example was conducted for each sample in duplicate (for the convenience of the experiment, samples were given branch numbers like control-1, control-2, E7070-1, E7070-2 and so on for distinction). Then, GeneChip (Affymetrix) system (Human Focus array) was used for analyzing changes in the gene expression induced by each compound.

Twenty-six ".cel" files obtained in this example (13 samples (a control + 12 compounds) x 2) were subjected to RMA method (robust multi-array average method (Biostatistics (2003), 4, 249-264)) for normal distribution at probe level, and then the logarithm value of the signal intensity at gene level was calculated. Next, the logarithm value of the signal intensity of the untreated group (control-1) was subtracted from the logarithm value of the signal intensity of the compound-treated group for each gene to obtain the logarithm value of the signal ratio of the compound-treated group to control-1. Then, cosine correlation coefficients were calculated as correlation coefficients between the experiments (Figure 5). Based on these correlation coefficients, hierarchical cluster analysis was performed according to UPGMA method (Unweighted Pair Group Method with Arithmetic mean method) (Figure 6). Control-2 was also subjected to similar calculation (Figures 7 and 8). The softwares used were R 2.0.1 (http://www.r-project.org/) and affy package 1.5.8 (http://www.bioconductor.org).

In Figures 5-8, "LY1" represents LY186641, "LY2" represents LY295501, "LY5" represents LY573636, "CAI" represents ethoxzolamide, "Cap" represents capsaicin, "MST" represents MST16, "Etop" represents etoposide, "TSA" represents trichostatin A, and "Kenp" represents kenpaullone. In Figures 6 and 8, "de hclust (*, "average")" is a command upon statistical analysis, showing that clustering analysis is conducted by R using the average value of the duplicate experiment data.

As a result of the analysis, E7070, E7820, LY186641, LY295501, LY573636 and CQS showed very similar genetic alterations for the HCT116 cells, and were found to be different from the profiles of any of the other compounds (MST16, etoposide, ethoxzolamide, capsaicin, trichostatin A and kenpaullone) (Figures 5-8). Thus, by this analysis, E7070, E7820, LY186641, LY295501, LY573636 and CQS were considered to have the same or similar action mechanisms, strongly suggesting that they give the same or similar genetic alterations and effects.

EXAMPLE 9: Experiment on cancer cell line panels

Human cancer cell panels from 36 cell lines were used to examine correlation of antiproliferative activities among E7820, E7070, CQS, LY186641 and LY295501. The 36 types of cancer cell lines used were DLD-1, HCT15, HCT116, HT29, SW480, SW620 and WiDr (which are human colon cancer cell lines), A427, A549, LX-1, NCI-H460, NCI-H522, PC-9 and PC-10 (which are human lung cancer cell lines), GT3TKB, HGC27, MKN1, MKN7, MKN28 and MKN74 (which are human gastric cancer cell lines), AsPC-1, KP-1, KP-4, MiaPaCaII, PANC-1 and SUIT-2 (which are human pancreas cancer cell lines), BSY-1, HBC5, MCF-7, MDA-MB-231, MDA-MB-435 and MDA-MB-468 (which are human breast cancer cell lines), and CCRF-CEM, HL60, K562 and MOLT-4 (which are human leukemia cell lines). All of the cells were cultured using RPMI-1640 media supplemented with 10% fetal bovine serum, 100 units/ml penicillin and 100 μg/ml streptomycin under the conditions of 5% $CO_2$ and 37°C (Table 8).

Table 8

**36 human cancer cell lines tested in 3-day MTT assays**

| Colon | Stomach | Breast |
|---|---|---|
| DLD-1 (1250/well, 16.8 h) | GT3TKB (2000/well, 21.1 h) | BSY-1 (2000/well, 46.1 h) |
| HCT15 (1500/well, 14.5 h) | HGC27 (1500/well, 14.6 h) | HBC5 (2000/well, 31.8 h) |
| HCT116 (1250/well, 13.4 h) | MKN1 (4000/well, 35.9 h) | MCF-7 (3000/well, 29.5 h) |
| HT29 (2500/well, 19.8 h) | MKN7 (3000/well, 37.4 h) | MDA-MB231 (2000/well, 21.6 h) |
| SW480 (3000/well, 19.5 h) | MKN28 (2000/well, 22.7 h) | MDA-MB-435 (3000/well, 24.4 h) |
| SW620 (2500/well, 17.3 h) | MKN74 (4000/well, 24.8 h) | MDA-MB-468 (3000/well, 34.2 h) |
| WiDr (2000/well, 18.9 h) | | |

(continued)

| Lung | Pancreas | Leukemia |
|---|---|---|
| **A427 (2500/well, 32.4 h)** | **AsPC-1 (2500/well, 28.4 h)** | **CCRF-CEM (1500/well, 27.2 h)** |
| **A549 (1250/well, 18.9 h)** | **KP-1 (2000/well, 24.8 h)** | **HL60 (1500/well, 29.5 h)** |
| **LX-1 (2000/well, 17.2 h)** | **KP-4 (2000/well, 16.7 h)** | **K562 (1500/well, 20.6 h)** |
| **NCI-H460 (1000/well, 13.6 h)** | **MiaPaCall (2500/well, 19.1 h)** | **MOLT-4 (1500/well, 22.3 h)** |
| **NCI-H522 (4000/well, 80.4 h)** | **PANC-1 (2500/well, 27.9 h)** | |
| **PC-9 (2000/well, 23.7 h)** | **SUIT-2 (2000/well, 15.6 h)** | |
| **PC-10 (2000/well, 24.0 h)** | | |

**Cell line (initial cell number, doubling time)**

Table 8 shows the types, seeded cell numbers and doubling times of the human cancer cell lines in the human cancer cell line panels.

The cells were seeded on a 96-well microplate (flat bottom) at the number indicated in Table 8 (50 μl/well). Twenty-four hours later, they were added with a 3-fold dilution series of each compound (50 μl/well). Seventy-two hours later, WST-8 (10 μl/well) was added and absorbance at 450 nm was determined. The 50% growth inhibitory concentrations to all of the 36 cancer cell lines were obtained by a least square method and their patterns were compared between the compounds. As the correlation index, Pearson's correlation coefficients were used (Paull, K. D. et al. Display and analysis of patterns of differential activity of drugs against human tumor cell lines: development of mean graph and COMPARE algorithm. J. Natl. Cancer Inst. 1989, 81, 1088-1092; Monks, A. et al. Feasibility of a high-flux anticancer drug screen using a diverse panel of cultured human tumor cell lines. J. Natl. Cancer Inst. 1991, 83, 757-766).

As a result, E7070, E7820, LY186641, LY295501 and CQS showed high correlation coefficients in antiproliferative activities against each cancer cell line (Table 9). Thus, by this analysis, E7070, E7820, LY186641, LY295501 and CQS were considered to have the same or similar action mechanisms, strongly suggesting that they give the same or similar genetic alterations and effects.

Table 9

| | E7070 | E7820 | CQS | LY186641 | LY295501 |
|---|---|---|---|---|---|
| E7070 | 1.00 | 0.98 | 0.97 | 0.93 | 0.80 |
| E7820 | 0.98 | 1.00 | 0.96 | 0.95 | 0.82 |
| CQS | 0.97 | 0.96 | 1.00 | 0.92 | 0.82 |
| LY186641 | 0.93 | 0.95 | 0.92 | 1.00 | 0.81 |
| LY295501 | 0.80 | 0.82 | 0.82 | 0.81 | 1.00 |

Table 9 shows correlation coefficients between the compounds (E7070, E7820, CQS, LY186641 and LY295501) on the human cancer cell line panels.

EXAMPLE 10: Cross-resistance in E7070-resistant cell line

An E7070-resistant cell line was used to assess the antiproliferative activities of E7820, LY186641, LY295501, LY-ASAP and CQS. HCT116-C9 was a substrain separated from human colon cancer-derived HCT116 (American Type Culture Collection, Manassas, VA, U.S.A.). This HCT116-C9 was cultured in the presence of E7070 while incrementally increasing the E7070 concentration, thereby obtaining E7070-resistant substrains HCT116-C9-C1 and HCT116-C9-C4 (Molecular Cancer Therapeutics, 2002, 1, 275-286).

Three cell lines, i.e., HCT116-C9, HCT116-C9-C1 and HCT116-C9-C4, were each seeded at 3000 cells/well onto a 96-well microplate (flat bottom) (50μl/well). Twenty-four hours later, they were added with a 3-fold dilution series of each compound (50 μl/well). Seventy-two hours later, the antiproliferative activities were assessed by MTT method (Mossmann T., J. Immunol. Methods, 1983, 65, 55-63). The 50% growth inhibitory concentrations to the cancer cells were obtained by a least square method.

As a result, the antiproliferative activity, i.e., IC50, of E7070 to HCT116-C9 (C9) was 0.127 μM. On the other hand, activities to HCT116-C9-C1 (C9C1) and HCT116-C9-C4 (C9C4) were IC50 = 31.9 μM and 26.9 μM, respectively, confirming that the antiproliferative activities of E7070 to C9C1 and C9C4 were remarkably low (Figure 9). The antiproliferative activities of E7820, CQS, LY186641, LY295501 and LY-ASAP to HCT116-C9 were IC50 = 0.080 μM, 1.73 μM, 33.6 μM, 10.9 μM and 1.63 μM, respectively while their activities to HCT116-C9-C1 were IC50 = 51.2 μM, 634 μM, 134 μM, 111 μM and 113 μM, respectively and their activities to HCT116-C9-C4 were IC50 = 52.8 μM, 517 μM, 138 μM, 110 μM and 90.3 μM, respectively. Therefore, the antiproliferative activities of E7820, CQS, LY186641, LY295501 and LY-ASAP to C9C1 and C9C4 were far lower than those to C9 (Figure 9). Thus, E7070, E7820, LY186641, LY295501, LY-ASAP and CQS were considered to have the same or similar action mechanisms, strongly suggesting that they give the same or similar genetic alterations and effects.

EXAMPLE 11: Cross-resistance in E7070-resistant cell line

In exactly the same manner as in Example 10, an E7070-resistant cell line was used to assess the antiproliferative activities of LY573636 as well as those of E7070.

As a result, the antiproliferative activities of E7070 to HCT116-C9-C1 and HCT116-C9-C4 (IC50 = 32.7 μM and 28.0 μM, respectively) were again confirmed to be remarkably lower than the activity to HCT116-C9 (IC50 = 0.127 μM) (Figure 10). The antiproliferative activities of LY573636 to HCT116-C9-C1 and HCT116-C9-C4 (IC50 = 264 μM and 240 μM, respectively) were also remarkably lower than the activity to HCT116-C9 (IC50 = 5.11 μM) (Figure 10). Thus, LY573636 was considered to have the same or similar action mechanism to that of E7070, strongly suggesting that it gives the same or similar genetic alteration and effect.

These results (Examples 7-11) confirmed that E7070, E7820, LY186641, LY295501, LY-ASAP, LY573636, CQS or a combination thereof give the same or similar genetic alterations and thus resulting in the same or similar actions and effects.

Accordingly, similar to E7820 (Examples 1-6), a sulfonamide compound, preferably E7070, LY186641, LY295501, LY-ASAP, LY573636, CQS or a combination thereof was found to show a remarkable anti-tumor activity and angiogenesis inhibiting activity upon combinational use with at least one compound selected from (i) a platinum complex, preferably Oxaliplatin or Cisplatin, (ii) a DNA-topoisomerase I inhibitor, preferably CPT-11, (iii) an antimetabolite, preferably Gemcitabine or Methotrexate, (iv) a microtubule inhibitor, preferably Paclitaxel and (v) an antibiotic, preferably Doxorubicin.

INDUSTRIAL APPLICABILITY

The present invention provides a pharmaceutical composition and a kit that show a remarkable anti-tumor activity and/or angiogenesis inhibiting activity, and a method for treating cancer and/or a method for inhibiting angiogenesis.

More specifically, the present invention provides a pharmaceutical composition and a kit that show a remarkable anti-tumor activity and/or angiogenesis inhibiting activity, and a method for treating cancer and/or a method for inhibiting angiogenesis, characterized in combining a sulfonamide compound (i.e., at least one compound selected from: (A) E7820; (B) a compound represented by General Formula (I), preferably LY186641 or LY295501; (C) a compound represented by General Formula (II), preferably LY-ASAP; (D) LY573636; and (E) CQS) with at least one substance selected from (i) a platinum complex, preferably Oxaliplatin or Cisplatin, (ii) a DNA-topoisomerase I inhibitor, preferably CPT-11, (iii) an antimetabolite, preferably Gemcitabine or Methotrexate, (iv) a microtubule inhibitor, preferably Paclitaxel and (v) an antibiotic, preferably Doxorubicin. The pharmaceutical composition, the kit and the method of the invention are useful for treating cancer or for inhibiting angiogenesis.

## Claims

1. A pharmaceutical composition comprising N-(3-cyano-4-methyl-1H-indole-7-yl)-3-cyanobenzenesulfonamide, a pharmacologically acceptable salt thereof or a solvate thereof in combination with at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof.

2. The pharmaceutical composition according to Claim 1, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

3. A pharmaceutical composition comprising a sulfonamide compound in combination with at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof,

wherein the sulfonamide compound is at least one compound selected from the group consisting of:

a compound represented by General Formula (I)

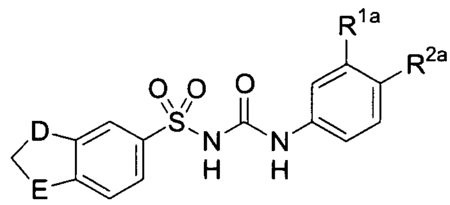

(I)

[wherein, E represents -O-, $-N(CH_3)-$, $-CH_2-$, $-CH_2CH_2-$ or $-CH_2O-$ D represents $-CH_2-$or -O-, $R^{1a}$ represents a hydrogen atom or a halogen atom, and $R^{2a}$ represents a halogen atom or a trifluoromethyl group];

a compound represented by General Formula (II)

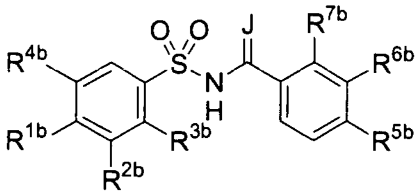

(II)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];

a compound represented by Formula (III)

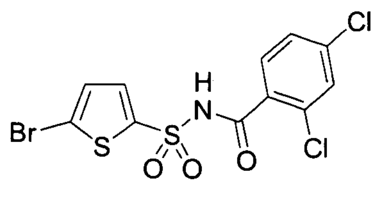

(III)

;

and

a compound represented by Formula (IV)

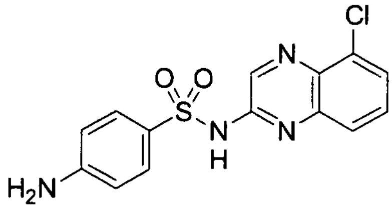 (IV)

or a pharmacologically acceptable salt thereof, or a solvate thereof.

4. The pharmaceutical composition according to Claim 3, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

5. The pharmaceutical composition according to Claim 3 or 4, wherein the sulfonamide compound is at least one compound selected from the group consisting of N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

6. The pharmaceutical composition according to Claim 3 or 4, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

7. The pharmaceutical composition according to Claim 3 or 4, wherein the sulfonamide compound is sodium salt of N-(2,4-dichlorobenzoyl)-5-bromothiophene-2-sulfonamide.

8. The pharmaceutical composition according to any one of Claims 1 to 7, wherein the pharmaceutical composition is used for treating cancer.

9. The pharmaceutical composition according to any one of Claims 1 to 7, wherein the pharmaceutical composition is used for inhibiting angiogenesis.

10. A kit comprising:

(a) at least one selected from the group consisting of a packaging container, an instruction and a package insert describing the combinational use of N-(3-cyano-4-methyl-1H-indole-7-yl)-3-cyanobenzenesulfonamide, a pharmacologically acceptable salt thereof or a solvate thereof and at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof; and
(b) a pharmaceutical composition comprising N-(3-cyano-4-methyl-1H-indole-7-yl)-3-cyanobenzenesulfonamide, a pharmacologically acceptable salt thereof or a solvate thereof.

11. The kit according to Claim 11, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

12. A kit comprising:

(a) at least one selected from the group consisting of a packaging container, an instruction and a package insert describing the combinational use of a sulfonamide compound and at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof; and
(b) a pharmaceutical composition comprising a sulfonamide compound,
wherein, the sulfonamide compound is at least one compound selected from the group consisting of:

a compound represented by General Formula (I)

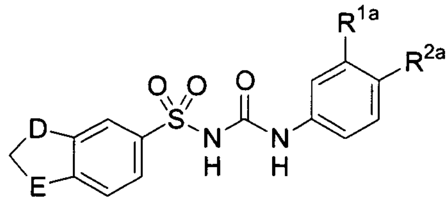

(I)

[wherein, E represents -O-, $-N(CH_3)-$, $-CH_2-$, $-CH_2CH_2-$ or $-CH_2O-$, D represents $-CH_2-$or -O-, $R^{1a}$ represents a hydrogen atom or a halogen atom, and $R^{2a}$ represents a halogen atom or a trifluoromethyl group];
a compound represented by General Formula (II)

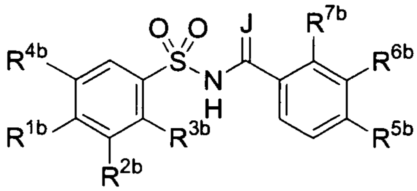

(II)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];
a compound represented by Formula (III)

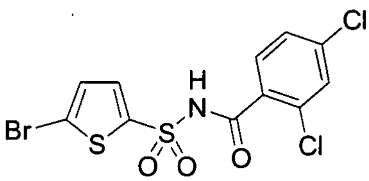

(III)

;

and
a compound represented by Formula (IV)

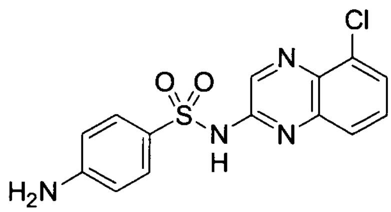 (IV)

or a pharmacologically acceptable salt thereof, or a solvate thereof.

**13.** The kit according to Claim 12, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**14.** The kit according to Claim 12 or 13, wherein the sulfonamide compound is at least one compound selected from the group consisting of N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

**15.** The kit according to Claim 12 or 13, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**16.** The kit according to Claim 12 or 13, wherein the sulfonamide compound is sodium salt of N-(2,4-dichlorobenzoyl)-5-bromothiophene-2-sulfonamide.

**17.** The kit according to any one of Claims 10-16, wherein the kit is used for treating cancer.

**18.** The kit according to any one of Claims 10-16, wherein the kit is used for inhibiting angiogenesis.

**19.** A kit comprising a set of a formulation comprising N-(3-cyano-4-methyl-1H-indole-7-yl)-3-cyanobenzenesulfonamide, a pharmacologically acceptable salt thereof or a solvate thereof and a formulation comprising at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof.

**20.** The kit according to Claim 19, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**21.** A kit comprising a set of a formulation comprising a sulfonamide compound and a formulation comprising at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof,
wherein, the sulfonamide compound is at least one compound selected from the group consisting of:

a compound represented by General Formula (I)

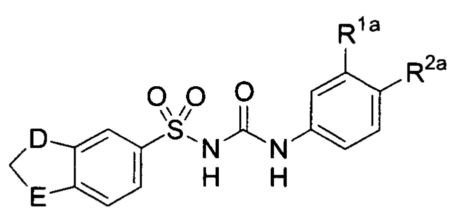 (I)

[wherein, E represents -O-, $-N(CH_3)-$, $-CH_2-$, $-CH_2CH_2-$ or $-CH_2O-$, D represents $-CH_2-$or -O-, $R^{1a}$ represents a hydrogen atom or a halogen atom, and $R^{2a}$ represents a halogen atom or a trifluoromethyl group];
a compound represented by General Formula (II)

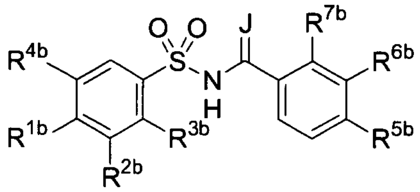

(II)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];
a compound represented by Formula (III)

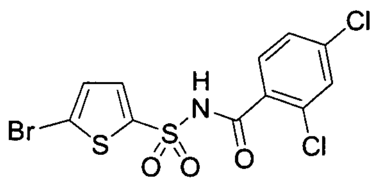

(III)

;

and
a compound represented by Formula (IV)

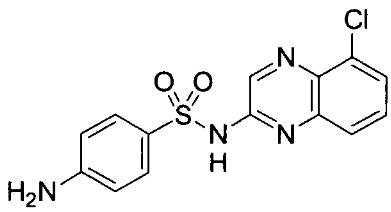

(IV)

or a pharmacologically acceptable salt thereof, or a solvate thereof.

22. The kit according to Claim 21, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**23.** The kit according to Claim 21 or 22, wherein the sulfonamide compound is at least one compound selected from the group consisting of N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

**24.** The kit according to Claim 21 or 22, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**25.** The kit according to Claim 21 or 22, wherein the sulfonamide compound is sodium salt of N-(2,4-dichlorobenzoyl)-5-bromothiophene-2-sulfonamide.

**26.** The kit according to any one of Claims 19-25, wherein the kit is used for treating cancer.

**27.** The kit according to any one of Claims 19-25, wherein the kit is used for inhibiting angiogenesis.

**28.** Use of N-(3-cyano-4-methyl-1H-indole-7-yl)-3-cyanobenzenesulfonamide, a pharmacologically acceptable salt thereof or a solvate thereof for producing a pharmaceutical composition in combination with at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof.

**29.** The use according to Claim 28, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**30.** Use of a sulfonamide compound for producing a pharmaceutical composition in combination with at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof, wherein the sulfonamide compound is at least one compound selected from the group consisting of:

a compound represented by General Formula (I)

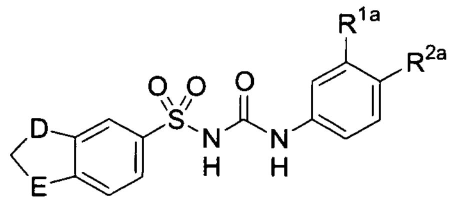

(I)

[wherein, E represents -O-, $-N(CH_3)-$, $-CH_2-$, $-CH_2CH_2-$ or $-CH_2O-$, D represents $-CH_2-$or -O-, $R^{1a}$ represents a hydrogen atom or a halogen atom, and $R^{2a}$ represents a halogen atom or a trifluoromethyl group];
a compound represented by General Formula (II)

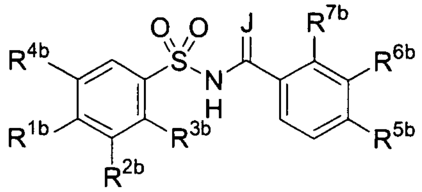

(II)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group,

$-CF_3$, $-OCF_3$, $-SCF_3$, an optionally substituted $C_1$-$C_4$ alkoxy carbonyl group, a nitro group, an azido group, -O $(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];

a compound represented by Formula (III)

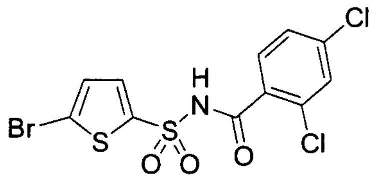

(III)

;

and

a compound represented by Formula (IV)

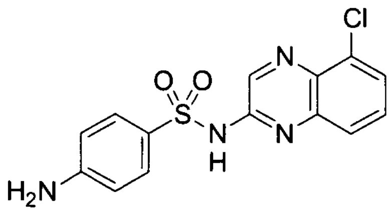

(IV)

or a pharmacologically acceptable salt thereof, or a solvate thereof.

**31.** The use according to Claim 30, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**32.** The use according to Claim 30 or 31, wherein the sulfonamide compound is at least one compound selected from the group consisting of N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

**33.** The use according to Claim 30 or 31, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**34.** The use according to Claim 30 or 31, wherein the sulfonamide compound is sodium salt of N-(2,4-dichlorobenzoyl)-5-bromothiophene-2-sulfonamide.

**35.** The use according to any one of Claims 28 to 34, wherein the pharmaceutical composition is a pharmaceutical

composition for treating cancer.

**36.** The use according to any one of Claims 28 to 34, wherein the pharmaceutical composition is a pharmaceutical composition for inhibiting angiogenesis.

**37.** A method for treating cancer and/or a method for inhibiting angiogenesis comprising administering N-(3-cyano-4-methyl-1H-indole-7-yl)-3-cyanobenzenesulfonamide, a pharmacologically acceptable salt thereof or a solvate thereof and at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof to a patient.

**38.** The method according to Claim 37, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**39.** A method for treating cancer and/or a method for inhibiting angiogenesis comprising administering a sulfonamide compound and at least one substance selected from the group consisting of a platinum complex, a DNA-topoisomerase I inhibitor, an antimetabolite, a microtubule inhibitor and an antibiotic, or a pharmacologically acceptable salt thereof or a solvate thereof to a patient,
wherein the sulfonamide compound is at least one compound selected from the group consisting of:

a compound represented by General Formula (I)

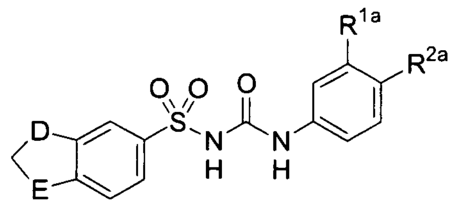

(I)

[wherein, E represents -O-, $-N(CH_3)-$, $-CH_2-$, $-CH_2CH_2-$ or $-CH_2O-$ D represents $-CH_2-$or -O-, $R^{1a}$ represents a hydrogen atom or a halogen atom, and $R^{2a}$ represents a halogen atom or a trifluoromethyl group];
a compound represented by General Formula (II)

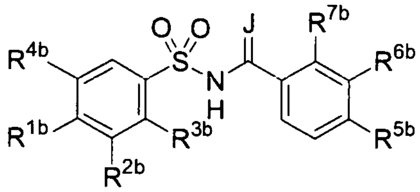

(II)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted

$C_1$-$C_6$ alkyl group or -$CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];
a compound represented by Formula (III)

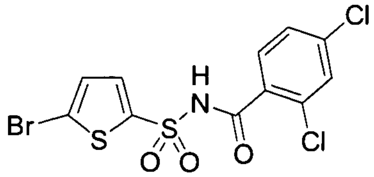

(III)

;

and
a compound represented by Formula (IV)

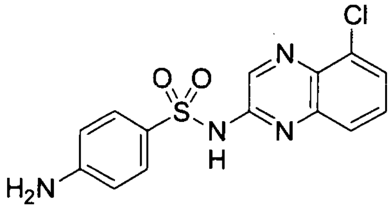

(IV)

or a pharmacologically acceptable salt thereof, or a solvate thereof.

**40.** The method according to Claim 39, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**41.** The method according to Claim 39 or 40, wherein the sulfonamide compound is at least one compound selected from the group consisting of N-[[(4-chlorophenyl)amhxo]carbonyl]-2,3-dihydro-1H-hxdene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

**42.** The method according to Claim 39 or 40, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**43.** The method according to Claim 39 or 40, wherein the sulfonamide compound is sodium salt of N-(2,4-dichlorobenzoyl)-5-bromothiophene-2-sulfonamide.

**44.** A pharmaceutical composition comprising N-(3-cyano-4-methyl-1H-indole-7-yl)-3-cyanobenzenesulfonamide, a pharmacologically acceptable salt thereof or a solvate thereof for administering to a patient in combination with at least one substance selected from the group consisting of a platinum complex, a DNA-topoisomerase I inhibitor, an antimetabolite, a microtubule inhibitor and an antibiotic.

**45.** The pharmaceutical composition according to Claim 44, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**46.** A pharmaceutical composition comprising a sulfonamide compound for administering to a patient in combination with at least one substance selected from the group consisting of a platinum complex, a DNA-topoisomerase I inhibitor, an antimetabolite, a microtubule inhibitor and an antibiotic,

wherein the sulfonamide compound is at least one compound selected from the group consisting of:

a compound represented by General Formula (I)

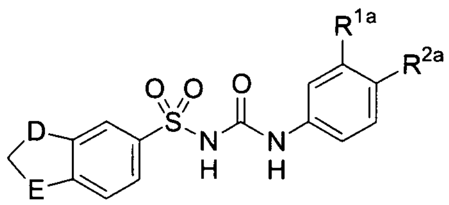

(I)

[wherein, E represents -O-, $-N(CH_3)-$, $-CH_2-$, $-CH_2CH_2-$ or $-CH_2O-$ D represents $-CH_2-$or -O-, $R^{1a}$ represents a hydrogen atom or a halogen atom, and $R^{2a}$ represents a halogen atom or trifluoromethyl group];
a compound represented by General Formula (II)

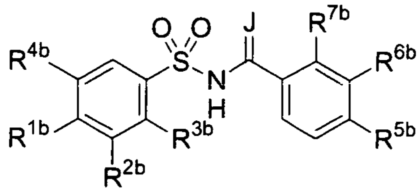

(II)

[wherein, E represents -O-, $-N(CH_3)-$, $-CH_2-$, $-CH_2CH_2-$ or $-CH_2O-$, D represents $-CH_2-$or -O-, $R^{1a}$ represents a hydrogen atom or a halogen atom, and $R^{2a}$ represents a halogen atom or a trifluoromethyl group];
a compound represented by General Formula (II)

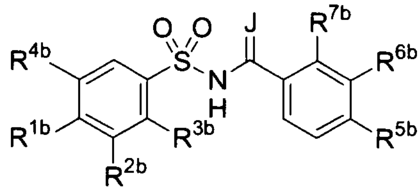

(II)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];
a compound represented by Formula (III)

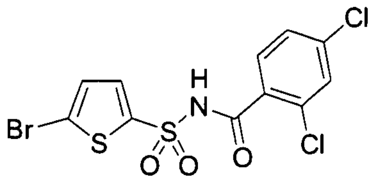

(III)

;

and

a compound represented by Formula (IV)

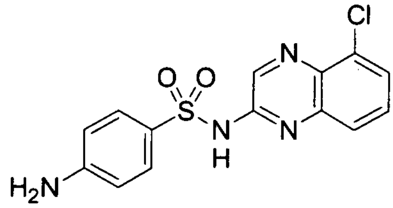

(IV)

or a pharmacologically acceptable salt thereof, or a solvate thereof.

**47.** The pharmaceutical composition according to Claim 46, wherein the group consisting of a platinum complex, a DNA-topoisomerase I inhibitor, an antimetabolite, a microtubule inhibitor and an antibiotic is a group consisting of Oxaliplatin, Cisplatin, CPT-11, Gemcitabine, Methotrexate, Paclitaxel and Doxorubicin.

**48.** The pharmaceutical composition according to Claim 46 or 47, wherein the sulfonamide compound is at least one compound selected from the group consisting of N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

**49.** The pharmaceutical composition according to Claim 46 or 47, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**50.** The pharmaceutical composition according to Claim 46 or 47, wherein the sulfonamide compound is sodium salt of N-(2,4-dichlorobenzoyl)-5-bromothiophene-2-sulfonamide.

**51.** The pharmaceutical composition according to any one of Claims 44-50, wherein the pharmaceutical composition is used for treating cancer.

**52.** The pharmaceutical composition according to any one of Claims 44-50, wherein the pharmaceutical composition is used for inhibiting angiogenesis.

Figure 1

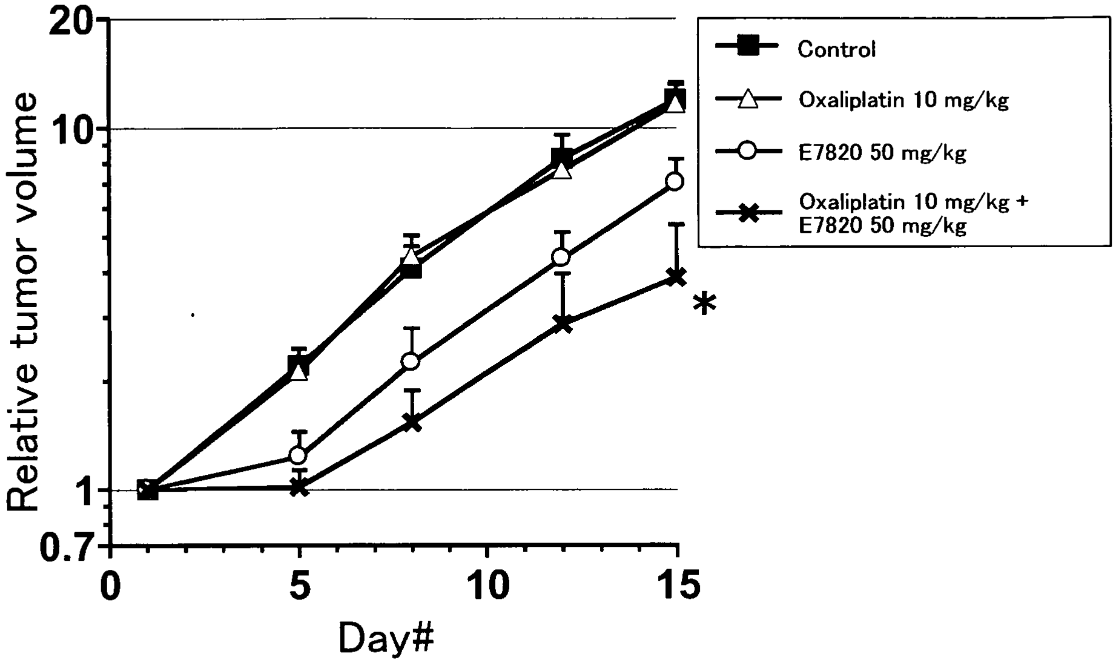
20
10
1
0.7
Relative tumor volume
0
5
10
15
Day#
Control
Oxaliplatin 10 mg/kg
E7820 50 mg/kg
Oxaliplatin 10 mg/kg +
E7820 50 mg/kg
*

Figure 2

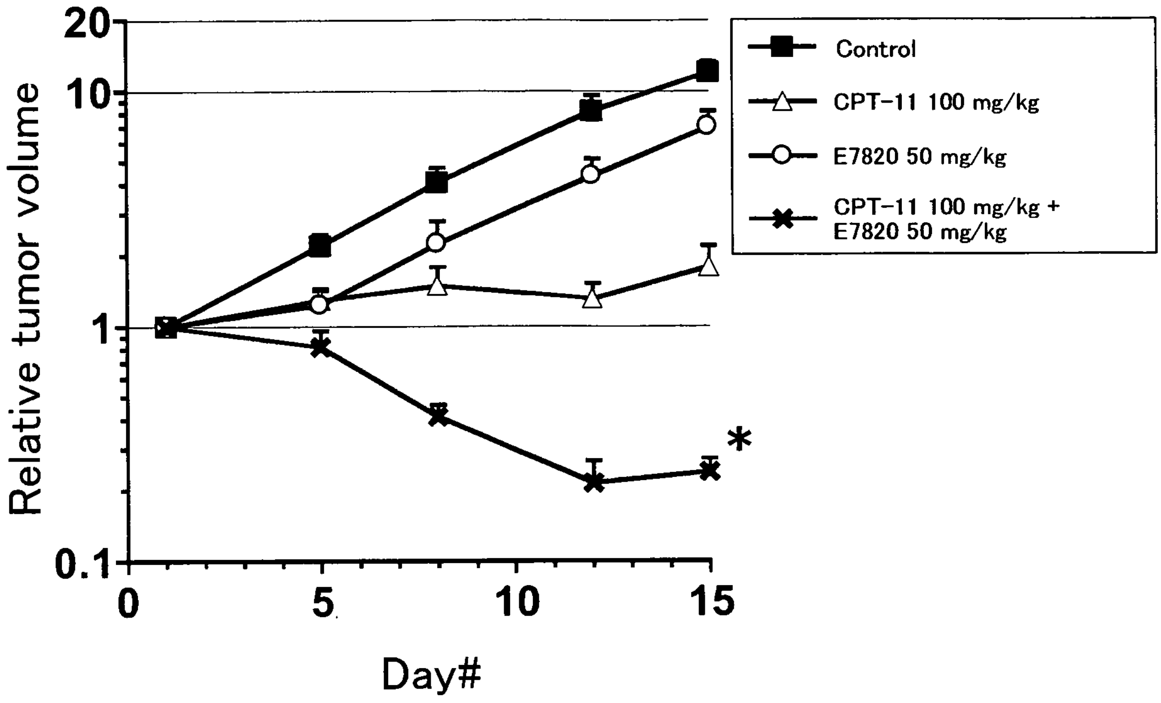
20
10
1
0.1
Relative tumor volume
0
5
10
15
Day#
Control
CPT-11 100 mg/kg
E7820 50 mg/kg
CPT-11 100 mg/kg +
E7820 50 mg/kg
*

Figure 3

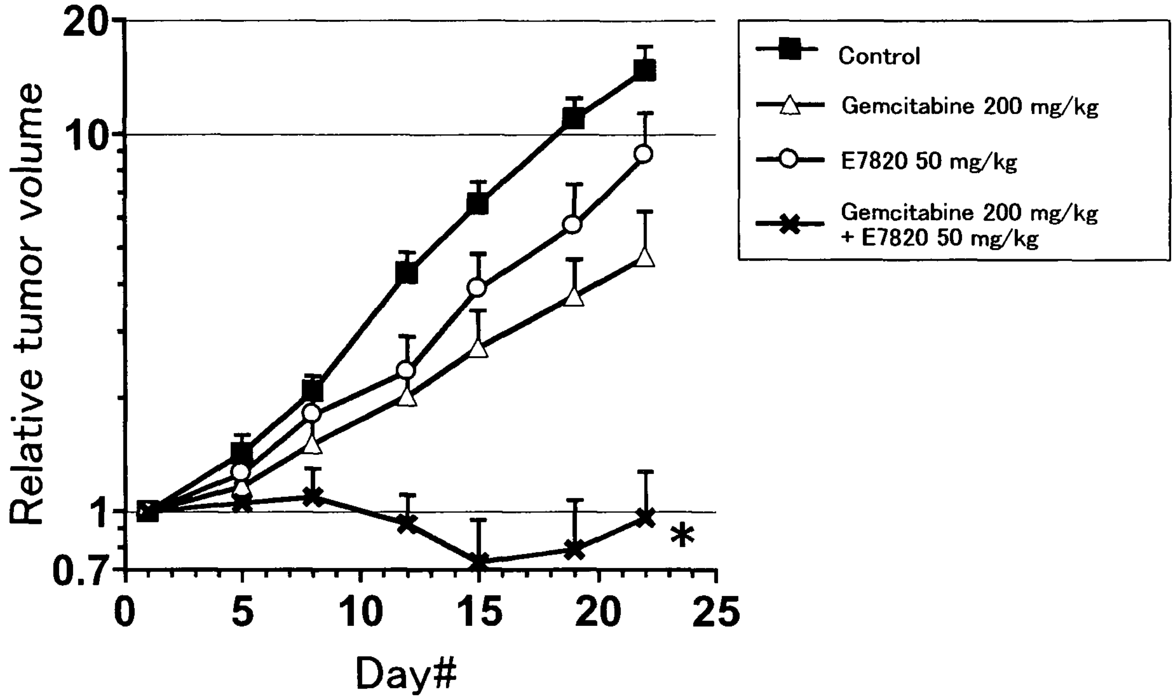
20
10
1
0.7
Relative tumor volume
0
5
10
15
20
25
Day#
Control
Gemcitabine 200 mg/kg
E7820 50 mg/kg
Gemcitabine 200 mg/kg
+ E7820 50 mg/kg
*

Figure 4

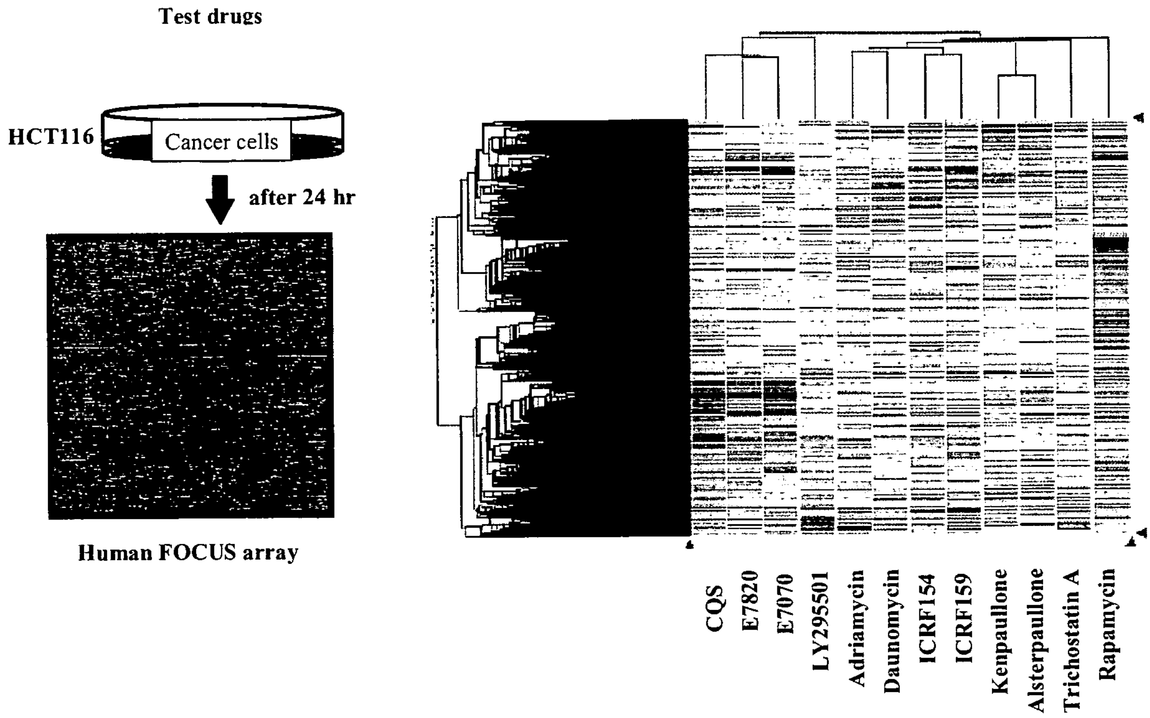
Test drugs
HCT116
Cancer cells
after 24 hr
Human FOCUS array
CQS
E7820
E7070
LY295501
Adriamycin
Daunomycin
ICRF154
ICRF159
Kenpaullone
Alsterpaullone
Trichostatin A
Rapamycin

Figure 5

| | E7070-1 | E7070-2 | E7820-1 | E7820-2 | CQS-1 | CQS-2 | LY1-1 | LY1-2 | LY2-1 | LY2-2 | LY5-1 | LY5-2 | CAI-1 | CAI-2 | Cap-1 | Cap-2 | MST-1 | MST-2 | Etop-1 | Etop-2 | TSA-1 | TSA-2 | Kenp-1 | Kenp-2 |
|---|---|---|---|---|---|---|---|---|---|---|---|---|---|---|---|---|---|---|---|---|---|---|---|---|
| E7070-1 | *1.00* | 0.95 | 0.87 | 0.87 | 0.87 | 0.87 | 0.83 | 0.83 | 0.86 | 0.86 | 0.88 | 0.88 | 0.16 | 0.17 | 0.11 | 0.11 | 0.35 | 0.35 | 0.16 | 0.17 | 0.31 | 0.31 | 0.32 | 0.32 |
| E7070-2 | 0.95 | *1.00* | 0.87 | 0.86 | 0.87 | 0.87 | 0.83 | 0.83 | 0.86 | 0.85 | 0.88 | 0.88 | 0.17 | 0.17 | 0.11 | 0.11 | 0.36 | 0.36 | 0.16 | 0.17 | 0.32 | 0.32 | 0.32 | 0.33 |
| E7820-1 | 0.87 | 0.87 | *1.00* | 0.96 | 0.90 | 0.90 | 0.88 | 0.88 | 0.83 | 0.82 | 0.88 | 0.88 | 0.09 | 0.09 | 0.07 | 0.07 | 0.26 | 0.26 | 0.08 | 0.08 | 0.24 | 0.24 | 0.20 | 0.21 |
| E7820-2 | 0.87 | 0.86 | 0.96 | *1.00* | 0.90 | 0.90 | 0.88 | 0.88 | 0.83 | 0.82 | 0.88 | 0.88 | 0.09 | 0.09 | 0.07 | 0.07 | 0.27 | 0.26 | 0.08 | 0.09 | 0.24 | 0.25 | 0.21 | 0.21 |
| CQS-1 | 0.87 | 0.87 | 0.90 | 0.90 | *1.00* | 0.96 | 0.87 | 0.87 | 0.84 | 0.83 | 0.90 | 0.90 | 0.13 | 0.14 | 0.10 | 0.10 | 0.37 | 0.36 | 0.16 | 0.16 | 0.32 | 0.32 | 0.29 | 0.29 |
| CQS-2 | 0.87 | 0.87 | 0.90 | 0.90 | 0.96 | *1.00* | 0.87 | 0.87 | 0.84 | 0.83 | 0.90 | 0.90 | 0.14 | 0.14 | 0.10 | 0.10 | 0.37 | 0.37 | 0.16 | 0.16 | 0.32 | 0.33 | 0.29 | 0.29 |
| LY1-1 | 0.83 | 0.83 | 0.88 | 0.88 | 0.87 | 0.87 | *1.00* | 0.97 | 0.82 | 0.81 | 0.89 | 0.89 | 0.25 | 0.25 | 0.24 | 0.24 | 0.30 | 0.30 | 0.12 | 0.13 | 0.25 | 0.26 | 0.25 | 0.25 |
| LY1-2 | 0.83 | 0.83 | 0.88 | 0.88 | 0.87 | 0.87 | 0.97 | *1.00* | 0.82 | 0.81 | 0.89 | 0.88 | 0.25 | 0.25 | 0.24 | 0.25 | 0.30 | 0.30 | 0.12 | 0.13 | 0.25 | 0.26 | 0.25 | 0.26 |
| LY2-1 | 0.86 | 0.86 | 0.83 | 0.83 | 0.84 | 0.84 | 0.82 | 0.82 | *1.00* | 0.94 | 0.85 | 0.85 | 0.20 | 0.20 | 0.14 | 0.15 | 0.35 | 0.35 | 0.17 | 0.18 | 0.34 | 0.34 | 0.32 | 0.32 |
| LY2-2 | 0.86 | 0.85 | 0.82 | 0.82 | 0.83 | 0.83 | 0.81 | 0.81 | 0.94 | *1.00* | 0.84 | 0.84 | 0.19 | 0.19 | 0.15 | 0.15 | 0.35 | 0.35 | 0.18 | 0.18 | 0.33 | 0.34 | 0.31 | 0.32 |
| LY5-1 | 0.88 | 0.88 | 0.88 | 0.88 | 0.90 | 0.90 | 0.89 | 0.89 | 0.85 | 0.84 | *1.00* | 0.97 | 0.20 | 0.20 | 0.15 | 0.15 | 0.39 | 0.39 | 0.21 | 0.22 | 0.34 | 0.34 | 0.31 | 0.32 |
| LY5-2 | 0.88 | 0.88 | 0.88 | 0.88 | 0.90 | 0.90 | 0.89 | 0.88 | 0.85 | 0.84 | 0.97 | *1.00* | 0.20 | 0.20 | 0.15 | 0.15 | 0.39 | 0.39 | 0.21 | 0.22 | 0.34 | 0.34 | 0.31 | 0.32 |
| CAI-1 | 0.16 | 0.17 | 0.09 | 0.09 | 0.13 | 0.14 | 0.25 | 0.25 | 0.20 | 0.19 | 0.20 | 0.20 | *1.00* | 0.98 | 0.75 | 0.75 | 0.38 | 0.38 | 0.34 | 0.34 | 0.25 | 0.25 | 0.25 | 0.25 |
| CAI-2 | 0.17 | 0.17 | 0.09 | 0.09 | 0.14 | 0.14 | 0.25 | 0.25 | 0.20 | 0.19 | 0.20 | 0.20 | 0.98 | *1.00* | 0.75 | 0.75 | 0.38 | 0.38 | 0.34 | 0.33 | 0.25 | 0.25 | 0.26 | 0.26 |
| Cap-1 | 0.11 | 0.11 | 0.07 | 0.07 | 0.10 | 0.10 | 0.24 | 0.24 | 0.14 | 0.15 | 0.15 | 0.15 | 0.75 | 0.75 | *1.00* | 0.99 | 0.35 | 0.34 | 0.31 | 0.31 | 0.19 | 0.19 | 0.14 | 0.14 |
| Cap-2 | 0.11 | 0.11 | 0.07 | 0.07 | 0.10 | 0.10 | 0.24 | 0.25 | 0.15 | 0.15 | 0.15 | 0.15 | 0.75 | 0.75 | 0.99 | *1.00* | 0.34 | 0.34 | 0.31 | 0.31 | 0.18 | 0.18 | 0.14 | 0.14 |
| MST-1 | 0.35 | 0.36 | 0.26 | 0.27 | 0.37 | 0.37 | 0.30 | 0.30 | 0.35 | 0.35 | 0.39 | 0.39 | 0.38 | 0.38 | 0.35 | 0.34 | *1.00* | 0.94 | 0.62 | 0.62 | 0.57 | 0.57 | 0.39 | 0.39 |
| MST-2 | 0.35 | 0.36 | 0.26 | 0.26 | 0.36 | 0.37 | 0.30 | 0.30 | 0.35 | 0.35 | 0.39 | 0.39 | 0.38 | 0.38 | 0.34 | 0.34 | 0.94 | *1.00* | 0.62 | 0.63 | 0.57 | 0.57 | 0.39 | 0.39 |
| Etop-1 | 0.16 | 0.16 | 0.08 | 0.08 | 0.16 | 0.16 | 0.12 | 0.12 | 0.17 | 0.18 | 0.21 | 0.21 | 0.34 | 0.34 | 0.31 | 0.31 | 0.62 | 0.62 | *1.00* | 0.96 | 0.45 | 0.45 | 0.24 | 0.24 |
| Etop-2 | 0.17 | 0.17 | 0.08 | 0.09 | 0.16 | 0.16 | 0.13 | 0.13 | 0.18 | 0.18 | 0.22 | 0.22 | 0.34 | 0.33 | 0.31 | 0.31 | 0.62 | 0.63 | 0.96 | *1.00* | 0.45 | 0.45 | 0.24 | 0.24 |
| TSA-1 | 0.31 | 0.32 | 0.24 | 0.24 | 0.32 | 0.32 | 0.25 | 0.25 | 0.34 | 0.33 | 0.34 | 0.34 | 0.25 | 0.25 | 0.19 | 0.18 | 0.57 | 0.57 | 0.45 | 0.45 | *1.00* | 0.93 | 0.34 | 0.34 |
| TSA-2 | 0.31 | 0.32 | 0.24 | 0.25 | 0.32 | 0.33 | 0.26 | 0.26 | 0.34 | 0.34 | 0.34 | 0.34 | 0.25 | 0.25 | 0.19 | 0.18 | 0.57 | 0.57 | 0.45 | 0.45 | 0.93 | *1.00* | 0.34 | 0.34 |
| Kenp-1 | 0.32 | 0.32 | 0.20 | 0.21 | 0.29 | 0.29 | 0.25 | 0.25 | 0.32 | 0.31 | 0.31 | 0.31 | 0.25 | 0.26 | 0.14 | 0.14 | 0.39 | 0.39 | 0.24 | 0.24 | 0.34 | 0.34 | *1.00* | 0.96 |
| Kenp-2 | 0.32 | 0.33 | 0.21 | 0.21 | 0.29 | 0.29 | 0.25 | 0.26 | 0.32 | 0.32 | 0.32 | 0.32 | 0.25 | 0.26 | 0.14 | 0.14 | 0.39 | 0.39 | 0.24 | 0.24 | 0.34 | 0.34 | 0.96 | *1.00* |

0.7<X<=1.0 (bold box)

0.4<X<=0.7 (dashed box)

0<X<=0.4 (thin box)

Figure 6

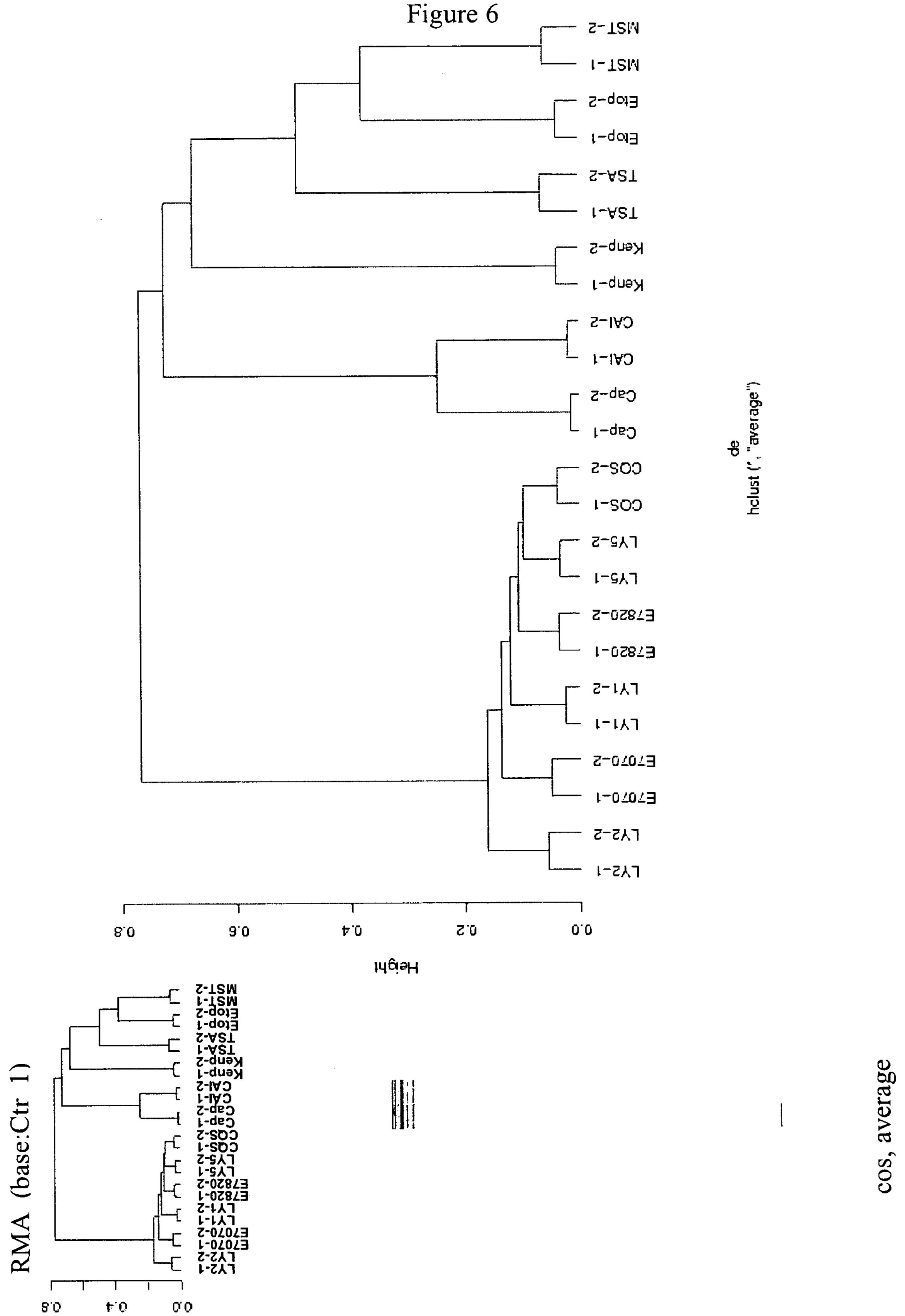
MST-2
MST-1
Etop-2
Etop-1
TSA-2
TSA-1
Kenp-2
Kenp-1
CAI-2
CAI-1
Cap-2
Cap-1
COS-2
COS-1
LY5-2
LY5-1
E7820-2
E7820-1
LY1-2
LY1-1
E7070-2
E7070-1
LY2-2
LY2-1
0.0
0.2
0.4
0.6
0.8
Height
de
hclust (*, "average")
RMA (base:Ctr 1)
cos, average

Figure 7

| | E7070-1 | E7070-2 | E7820-1 | E7820-2 | CQS-1 | CQS-2 | LY1-1 | LY1-2 | LY2-1 | LY2-2 | LY5-1 | LY5-2 | CAI-1 | CAI-2 | Cap-1 | Cap-2 | MST-1 | MST-2 | Etop-1 | Etop-2 | TSA-1 | TSA-2 | Kenp-1 | Kenp-2 |
|---|---|---|---|---|---|---|---|---|---|---|---|---|---|---|---|---|---|---|---|---|---|---|---|---|
| E7070-1 | *1.00* | 0.94 | 0.87 | 0.87 | 0.86 | 0.86 | 0.81 | 0.81 | 0.84 | 0.83 | 0.86 | 0.86 | 0.04 | 0.04 | 0.03 | 0.04 | 0.24 | 0.23 | 0.06 | 0.06 | 0.22 | 0.22 | 0.19 | 0.19 |
| E7070-2 | 0.94 | *1.00* | 0.86 | 0.86 | 0.85 | 0.85 | 0.81 | 0.81 | 0.84 | 0.83 | 0.86 | 0.86 | 0.04 | 0.04 | 0.03 | 0.03 | 0.24 | 0.23 | 0.05 | 0.06 | 0.22 | 0.22 | 0.19 | 0.20 |
| E7820-1 | 0.87 | 0.86 | *1.00* | 0.96 | 0.90 | 0.90 | 0.86 | 0.86 | 0.81 | 0.80 | 0.87 | 0.87 | 0.00 | 0.00 | 0.02 | 0.03 | 0.22 | 0.22 | 0.03 | 0.04 | 0.21 | 0.22 | 0.12 | 0.12 |
| E7820-2 | 0.87 | 0.86 | 0.96 | *1.00* | 0.90 | 0.90 | 0.85 | 0.85 | 0.81 | 0.80 | 0.87 | 0.87 | 0.00 | 0.00 | 0.02 | 0.03 | 0.22 | 0.21 | 0.03 | 0.04 | 0.21 | 0.22 | 0.12 | 0.13 |
| CQS-1 | 0.86 | 0.85 | 0.90 | 0.90 | *1.00* | 0.96 | 0.85 | 0.85 | 0.82 | 0.81 | 0.89 | 0.88 | 0.02 | 0.02 | 0.03 | 0.03 | 0.28 | 0.27 | 0.07 | 0.07 | 0.25 | 0.25 | 0.17 | 0.18 |
| CQS-2 | 0.86 | 0.85 | 0.90 | 0.90 | 0.96 | *1.00* | 0.86 | 0.85 | 0.82 | 0.81 | 0.89 | 0.89 | 0.02 | 0.02 | 0.03 | 0.04 | 0.28 | 0.27 | 0.07 | 0.07 | 0.25 | 0.25 | 0.17 | 0.18 |
| LY1-1 | 0.81 | 0.81 | 0.86 | 0.85 | 0.85 | 0.86 | *1.00* | 0.97 | 0.80 | 0.79 | 0.88 | 0.88 | 0.17 | 0.17 | 0.19 | 0.19 | 0.20 | 0.20 | 0.04 | 0.04 | 0.17 | 0.17 | 0.15 | 0.16 |
| LY1-2 | 0.81 | 0.81 | 0.86 | 0.85 | 0.85 | 0.85 | 0.97 | *1.00* | 0.80 | 0.79 | 0.87 | 0.87 | 0.17 | 0.17 | 0.19 | 0.20 | 0.20 | 0.20 | 0.03 | 0.04 | 0.17 | 0.17 | 0.15 | 0.16 |
| LY2-1 | 0.84 | 0.84 | 0.81 | 0.81 | 0.82 | 0.82 | 0.80 | 0.80 | *1.00* | 0.93 | 0.83 | 0.83 | 0.09 | 0.09 | 0.07 | 0.08 | 0.23 | 0.23 | 0.07 | 0.08 | 0.24 | 0.24 | 0.20 | 0.20 |
| LY2-2 | 0.83 | 0.83 | 0.80 | 0.80 | 0.81 | 0.81 | 0.79 | 0.79 | 0.93 | *1.00* | 0.82 | 0.82 | 0.08 | 0.08 | 0.07 | 0.08 | 0.23 | 0.22 | 0.07 | 0.07 | 0.23 | 0.24 | 0.19 | 0.20 |
| LY5-1 | 0.86 | 0.86 | 0.87 | 0.87 | 0.89 | 0.89 | 0.88 | 0.87 | 0.83 | 0.82 | *1.00* | 0.96 | 0.09 | 0.09 | 0.08 | 0.08 | 0.28 | 0.28 | 0.11 | 0.12 | 0.24 | 0.24 | 0.19 | 0.20 |
| LY5-2 | 0.86 | 0.86 | 0.87 | 0.87 | 0.88 | 0.89 | 0.88 | 0.87 | 0.83 | 0.82 | 0.96 | *1.00* | 0.09 | 0.09 | 0.07 | 0.08 | 0.28 | 0.28 | 0.11 | 0.12 | 0.24 | 0.24 | 0.19 | 0.20 |
| CAI-1 | 0.04 | 0.04 | 0.00 | 0.00 | 0.02 | 0.02 | 0.17 | 0.17 | 0.09 | 0.08 | 0.09 | 0.09 | *1.00* | 0.98 | 0.74 | 0.74 | 0.26 | 0.26 | 0.25 | 0.25 | 0.13 | 0.13 | 0.14 | 0.14 |
| CAI-2 | 0.04 | 0.04 | 0.00 | 0.00 | 0.02 | 0.02 | 0.17 | 0.17 | 0.09 | 0.08 | 0.09 | 0.09 | 0.98 | *1.00* | 0.74 | 0.74 | 0.26 | 0.26 | 0.25 | 0.25 | 0.13 | 0.13 | 0.15 | 0.15 |
| Cap-1 | 0.03 | 0.03 | 0.02 | 0.02 | 0.03 | 0.03 | 0.19 | 0.19 | 0.07 | 0.07 | 0.08 | 0.07 | 0.74 | 0.74 | *1.00* | 0.99 | 0.28 | 0.28 | 0.26 | 0.26 | 0.12 | 0.12 | 0.06 | 0.06 |
| Cap-2 | 0.04 | 0.03 | 0.03 | 0.03 | 0.03 | 0.04 | 0.19 | 0.20 | 0.08 | 0.08 | 0.08 | 0.08 | 0.74 | 0.74 | 0.99 | *1.00* | 0.28 | 0.28 | 0.26 | 0.25 | 0.11 | 0.12 | 0.06 | 0.06 |
| MST-1 | 0.24 | 0.24 | 0.22 | 0.22 | 0.28 | 0.28 | 0.20 | 0.20 | 0.23 | 0.23 | 0.28 | 0.28 | 0.26 | 0.26 | 0.28 | 0.28 | *1.00* | 0.92 | 0.57 | 0.57 | 0.50 | 0.50 | 0.24 | 0.25 |
| MST-2 | 0.23 | 0.23 | 0.22 | 0.21 | 0.27 | 0.27 | 0.20 | 0.20 | 0.23 | 0.22 | 0.28 | 0.28 | 0.26 | 0.26 | 0.28 | 0.28 | 0.92 | *1.00* | 0.57 | 0.57 | 0.50 | 0.50 | 0.24 | 0.25 |
| Etop-1 | 0.06 | 0.05 | 0.03 | 0.03 | 0.07 | 0.07 | 0.04 | 0.03 | 0.07 | 0.07 | 0.11 | 0.11 | 0.25 | 0.25 | 0.26 | 0.26 | 0.57 | 0.57 | *1.00* | 0.96 | 0.39 | 0.39 | 0.12 | 0.13 |
| Etop-2 | 0.06 | 0.06 | 0.04 | 0.04 | 0.07 | 0.07 | 0.04 | 0.04 | 0.08 | 0.07 | 0.12 | 0.12 | 0.25 | 0.25 | 0.26 | 0.25 | 0.57 | 0.57 | 0.96 | *1.00* | 0.39 | 0.39 | 0.12 | 0.13 |
| TSA-1 | 0.22 | 0.22 | 0.21 | 0.21 | 0.25 | 0.25 | 0.17 | 0.17 | 0.24 | 0.23 | 0.24 | 0.24 | 0.13 | 0.13 | 0.12 | 0.11 | 0.50 | 0.50 | 0.39 | 0.39 | *1.00* | 0.93 | 0.21 | 0.22 |
| TSA-2 | 0.22 | 0.22 | 0.22 | 0.22 | 0.25 | 0.25 | 0.17 | 0.17 | 0.24 | 0.24 | 0.24 | 0.24 | 0.13 | 0.13 | 0.12 | 0.12 | 0.50 | 0.50 | 0.39 | 0.39 | 0.93 | *1.00* | 0.22 | 0.22 |
| Kenp-1 | 0.19 | 0.19 | 0.12 | 0.12 | 0.17 | 0.17 | 0.15 | 0.15 | 0.20 | 0.19 | 0.19 | 0.19 | 0.14 | 0.15 | 0.06 | 0.06 | 0.24 | 0.24 | 0.12 | 0.12 | 0.21 | 0.22 | *1.00* | 0.95 |
| Kenp-2 | 0.19 | 0.20 | 0.12 | 0.13 | 0.18 | 0.18 | 0.16 | 0.16 | 0.20 | 0.20 | 0.20 | 0.20 | 0.14 | 0.15 | 0.06 | 0.06 | 0.25 | 0.25 | 0.13 | 0.13 | 0.22 | 0.22 | 0.95 | *1.00* |

0.7<X<=1.0 (solid bold box)

0.4<X<=0.7 (dashed box)

0<X<=0.4 (thin box)

Figure 8

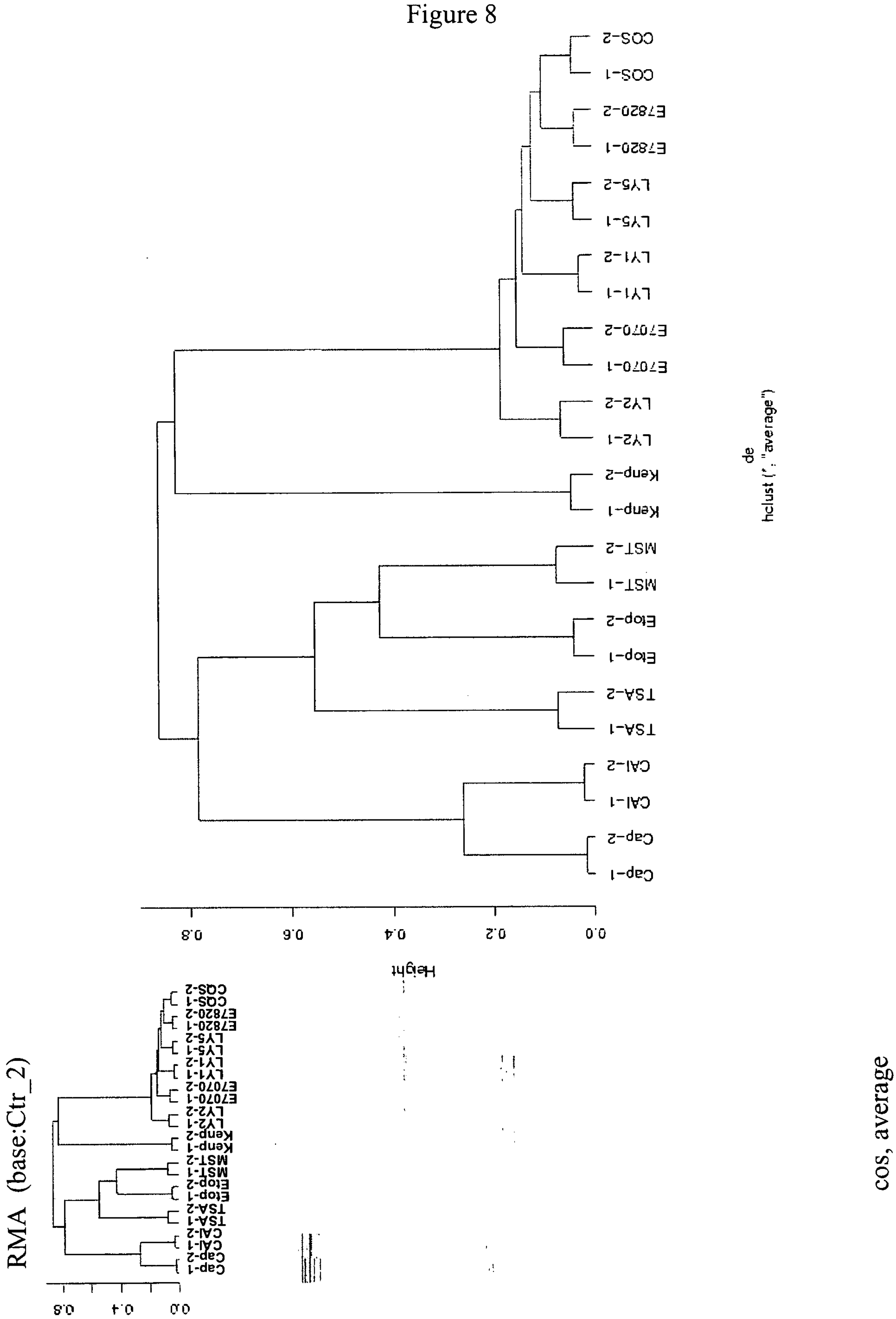
RMA (base:Ctr_2)
cos, average
Height
de
hclust (*, "average")
COS-2
COS-1
E7820-2
E7820-1
LY5-2
LY5-1
LY1-2
LY1-1
E7070-2
E7070-1
LY2-2
LY2-1
Kenp-2
Kenp-1
MST-2
MST-1
Etop-2
Etop-1
TSA-2
TSA-1
CAI-2
CAI-1
Cap-2
Cap-1
0.0
0.2
0.4
0.6
0.8

Figure 9

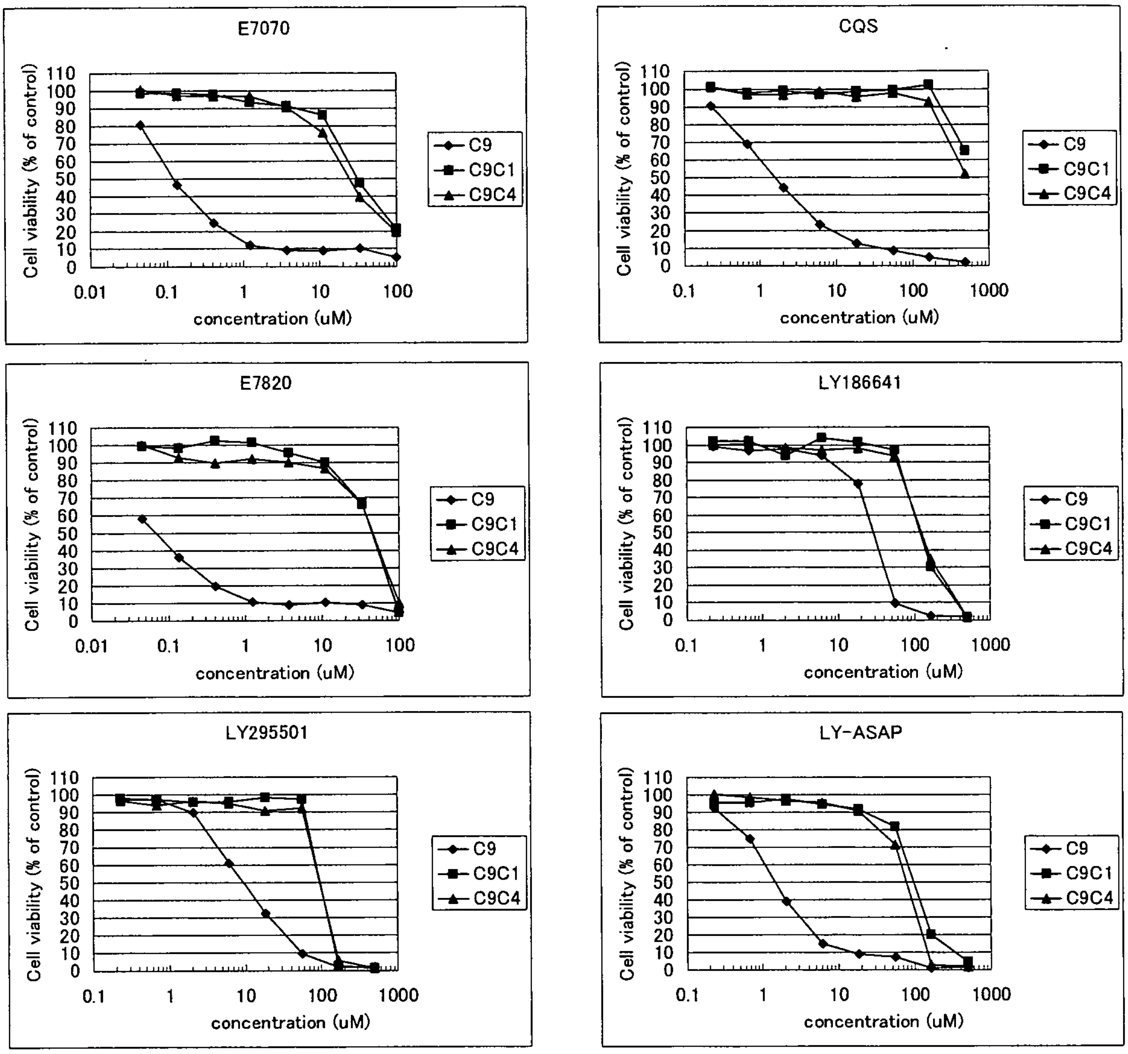
E7070
CQS
E7820
LY186641
LY295501
LY-ASAP
Cell viability (% of control)
concentration (uM)
C9
C9C1
C9C4
0.01
0.1
1
10
100
1000
0
10
20
30
40
50
60
70
80
90
100
110

Figure 10

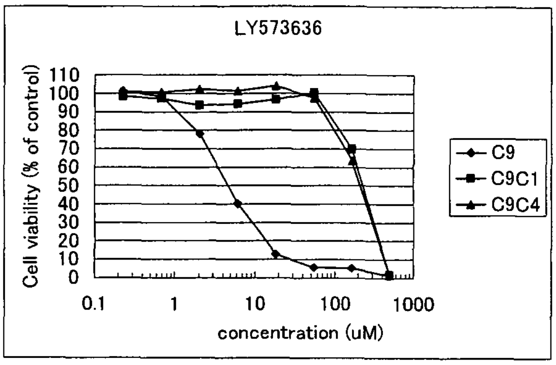
LY573636
Cell viability (% of control)
110
100
90
80
70
60
50
40
30
20
10
0
0.1
1
10
100
1000
concentration (uM)
C9
C9C1
C9C4

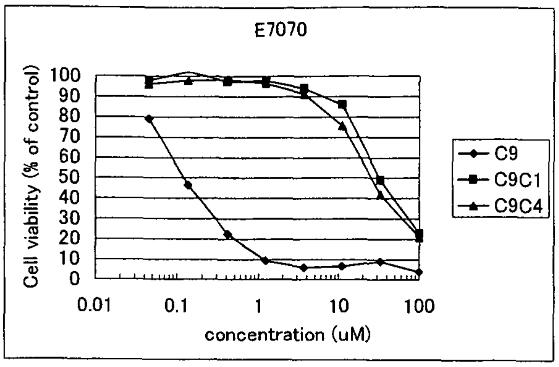
E7070
Cell viability (% of control)
100
90
80
70
60
50
40
30
20
10
0
0.01
0.1
1
10
100
concentration (uM)
C9
C9C1
C9C4

## INTERNATIONAL SEARCH REPORT

International application No.
PCT/JP2006/304219

A. CLASSIFICATION OF SUBJECT MATTER
***A61K31/404***(2006.01), ***A61K31/18***(2006.01), ***A61K31/343***(2006.01), ***A61K31/381***(2006.01), ***A61K31/4741***(2006.01), ***A61K31/498***(2006.01), ***A61K31/7068***(2006.01), ***A61K33/24***(2006.01), ***A61K45/00***(2006.01), ***A61P9/00***(2006.01),

B. FIELDS SEARCHED

Minimum documentation searched (classification system followed by classification symbols)
A61K31/18, A61K31/343, A61K31/381, A61K31/404, A61K31/4741, A61K31/498, A61K31/7068, A61K33/24, A61K45/00, A61P9/00, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br><br><br>Y | WO 2003/074045 A1 (EISAI CO., LTD.), 12 September, 2003 (12.09.03), Pages 21, 24 to 26<br>& AU 2003211594 A1 & EP 1481678 A1<br>& US 2005/119303 A1 & JP 2003-572563 A | 1,8-10, 17-19,26-28, 35,36,44,45, 51,52<br>2,11,20,29 |
| Y | WO 2002/36117 A1 (EISAI CO., LTD.), 10 May, 2002 (10.05.02), Full text<br>& AU 200210993 A & EP 1331005 A1<br>& KR 2003046475 A & US 2003/215523 A1<br>& US 2004/002505 A1 & JP 2002-538929 A<br>& CN 1473041 A & US 2004/224972 A1<br>& NZ 524975 A | 1,2,8-11, 17-20,26-29, 35,36,44,45, 51,52 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>10 May, 2006 (10.05.06) | Date of mailing of the international search report<br>23 May, 2006 (23.05.06) |
| Name and mailing address of the ISA/<br>Japanese Patent Office<br><br>Facsimile No. | Authorized officer<br><br><br>Telephone No. |

Form PCT/ISA/210 (second sheet) (April 2005)

**INTERNATIONAL SEARCH REPORT**

International application No.
PCT/JP2006/304219

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2002-533416 A (SEARLE & CO G D),<br>08 October, 2002 (08.10.02),<br>Full text<br>& WO 2000/38730 A2 & AU 200023805 A<br>& NO 200103155 A & EP 1140192 A2<br>& BR 9916518 A & HU 200104814 A2<br>& CZ 200102320 A3 & US 2003/203956 A1 | 1,2,8-11,<br>17-20,26-29,<br>35,36,44,45,<br>51,52 |

Form PCT/ISA/210 (continuation of second sheet) (April 2005)

**INTERNATIONAL SEARCH REPORT**

International application No.
PCT/JP2006/304219

Continuation of A. CLASSIFICATION OF SUBJECT MATTER
(International Patent Classification (IPC))

*A61P35/00*(2006.01)

(According to International Patent Classification (IPC) or to both national classification and IPC)

Form PCT/ISA/210 (extra sheet) (April 2005)

**INTERNATIONAL SEARCH REPORT**

International application No.
PCT/JP2006/304219

**Box No. II Observations where certain claims were found unsearchable (Continuation of item 2 of first sheet)**

This international search report has not been established in respect of certain claims under Article 17(2)(a) for the following reasons:

1. [X] Claims Nos.: 37-43
because they relate to subject matter not required to be searched by this Authority, namely:
Claims 37 to 43 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.:
Claims 1, 2, 10, 11, 19, 20, 28, 29, 44 and 45 and part of claims 8, 9, 17, 18, 26, 27, 35, 36, 51 and 52

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

INTERNATIONAL SEARCH REPORT

International application No.
PCT/JP2006/304219

Continuation of Box No. III of continuation of first sheet (2)

(Box No. III Continuation of observations where unity of invention is lacking)

Claims 1, 2, 10, 11, 19, 20, 28, 29, 44 and 45 and part of claims 8, 9, 17, 18, 26, 27, 35, 36, 51 and 52 relate to a combination of N-(3-cyano-4-methyl-1H-indol-7-yl)-3-cyanobenzenesulfonamide with at least one substance selected from a platinum complex compound, a DNA-topoisomerase I inhibitor, a metabolic antagonist, a microtubule inhibitor and an antibiotic.

Part of claims 3-9, 12-15, 17-18, 21-24, 26-27, 30-33, 35-36, 46-49 and 51-52 relates to a combination of a sulfonamide compound represented by the general formula (I) with at least one substance selected from a platinum complex compound, a DNA-topoisomerase I inhibitor, a metabolic antagonist, a microtubule inhibitor and an antibiotic.

Part of claims 3-9, 12-15, 17-18, 21-24, 26-27, 30-33, 35-36, 46-49 and 51-52 and claims 16, 25, 34 and 50 relate to a combination of a sulfonamide compound represented by the general formula (II) with at least one substance selected from a platinum complex compound, a DNA-topoisomerase I inhibitor, a metabolic antagonist, a microtubule inhibitor and an antibiotic.

Part of claims 3-9, 12-14, 17-18, 21-23, 26-27, 30-32, 35-36, 46-48 and 51-52 relates to a combination of a sulfonamide compound represented by the general formula (III) with at least one substance selected from a platinum complex compound, a DNA-topoisomerase I inhibitor, a metabolic antagonist, a microtubule inhibitor and an antibiotic.

Part of claims 3-9, 12-14, 17-18, 21-23, 26-27, 30-32, 35-36, 46-48 and 51-52 relates to a combination of a sulfonamide compound represented by the general formula (IV) with at least one substance selected from a platinum complex compound, a DNA-topoisomerase I inhibitor, a metabolic antagonist, a microtubule inhibitor and an antibiotic.

It is recognized that N-(3-cyano-4-methyl-1H-indol-7-yl)-3-cyanobenzenesulfonamide and sulfonamide compounds represented by the general formulae (I), (II), (III) and (IV) have a cyclic sulfonamide as a common chemical structure, however, a pharmaceutical composition comprising a compound with the basic skeleton in the compounds and another agent in combination is publicly known by the document (WO 2002/36117 A1 (EISAI CO LTD) 2002. 05. 10) and the like. Thus, this common matter is not considered to be a special technical feature.

Further, there is no other common matter, which is common to all claims and considered to be a special technical feature, therefore, the number of the inventions included in this application is at least 5.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0050395 A
- EP 0222475 A
- WO 02098848 A
- WO 03035629 A
- EP 0222475 A1
- EP 0555036 A2
- DE 4115559
- EP 193936 A
- US 6503943 B
- EP 328274 A
- WO 0010543 A
- US 5744497 A
- US 5004758 A
- JP 5059061 A
- JP 59051288 A
- JP 59051289 A
- WO 9529919 A
- WO 00053607 A
- WO 9530682 A
- EP 536939 A


### Non-patent literature cited in the description


- **SCHENA M ; SHALON D ; DAVIS RW ; BROWN PO.** *Science,* 1995, vol. 270, 467-70
- **LOCKHART, D.J. ; DONG, H. ; BYRNE, M.C. ; FOLLETTIE, M.T. ; GALLO, M.V. ; CHEE, M.S. ; MITTMANN, M. ; WANG C. ; KOBAYASHI, M. ; HORTON, H.** *Nature Biotechnology,* 1996, vol. 14, 1675-1680
- **RHEE CH ; RUAN S ; CHEN S ; CHENCHIK A ; LEVIN VA ; YUNG AW ; FULLER GN ; ZHANG W.** *Oncol Rep,* 1999, vol. 6, 393-401
- **ZIMMERMANN J ; ERDMANN D ; LALANDE I ; GROSSENBACHER R ; NOORANI M ; FURST P.** *Oncogene,* 2000, vol. 19, 2913-20
- **KUDOH K ; RAMANNA M ; RAVATN R ; ELKAHLOUN AG ; BITTNER ML ; MELTZER PS ; TRENT JM ; DALTON WS ; CHIN KV.** *Cancer Res,* 2000, 4161-6
- **ROSS DT ; SCHERF U ; EISEN MB ; PEROU CM ; REES C ; SPELLMAN P ; IYER V ; JEFFREY SS ; VAN DE RIJN M ; WALTHAM M.** *Nat Genet,* 2000, vol. 24, 227-35
- **SCHERF U ; ROSS DT ; WALTHAM M ; SMITH LH ; LEE JK ; TANABE L ; KOHN KW ; REINHOLD WC ; MYERS TG ; ANDREWS DT.** *Nat Genet,* 2000, vol. 24, 236-44
- *J. Am. Chem. Soc.,* 1947, vol. 71, 6-10
- *Journal of Medicinal Chemistry,* 1986, vol. 29 (11), 2358-63
- **CHOU et al.** *Adv. Enzyme Regul.,* 1984, vol. 22, 27-55
- **KIM R ; HIRABAYASHI N ; NISHIYAMA M et al.** Experimental studies on biochemical modulation targeting topoisomerase I and II in human tumor xenografts in nude mice. *Int J Cancer.,* 1992, vol. 50, 760-6
- **WHITACRE CM ; ZBOROWSKA E ; GORDON NH et al.** Topotecan increases topoisomerase II alpha levels and sensitivity to treatment with etoposide in schedule-dependent process. *Cancer Res.,* 1997, vol. 57, 1425-8
- *Biostatistics,* 2003, vol. 4, 249-264
- **PAULL, K. D. et al.** Display and analysis of patterns of differential activity of drugs against human tumor cell lines: development of mean graph and COMPARE algorithm. *J. Natl. Cancer Inst.,* 1989, vol. 81, 1088-1092
- **MONKS, A. et al.** Feasibility of a high-flux anticancer drug screen using a diverse panel of cultured human tumor cell lines. *J. Natl. Cancer Inst.,* 1991, vol. 83, 757-766
- *Molecular Cancer Therapeutics,* 2002, vol. 1, 275-286
- **MOSSMANN T.** *J. Immunol. Methods,* 1983, vol. 65, 55-63